# EUROPEAN PATENT SPECIFICATION

(11) **EP 3 510 170 B1**
(45) Date of publication and mention of the grant of the patent: **13.10.2021**
(21) Application number: 17771999.4
(22) Date of filing: 12.09.2017
(51) Int. Cl.: C12Q 1/6855

(54) **CONVERTIBLE ADAPTERS**
UMWANDELBARE ADAPTER
ADAPTATEURS CONVERTIBLES

(30) Priority: 12.09.2016 EP 16188292
(43) Date of publication of application: 17.07.2019
(73) Proprietor: Technische Hochschule Wildau, 15745 Wildau (DE)
(72) Inventor: GLÖKLER, Jörn, 10783 Berlin (DE); FROHME, Marcus, 15711 Königs Wusterhausen (DE)
(74) Representative: Schulz Junghans Patentanwälte PartGmbB
(86) International application number: PCT/EP2017/072862
(87) International publication number: WO 2018/046752

(56) References cited:
- EP-A1- 2 272 976
- WO-A1-2013/059746

## Description

The present invention is in the field of biochemistry and diagnostics. It is more particularly in the field of molecular biology and generation of nucleic acid libraries and more specifically relates to generation of nucleic acid library templates for sequencing.

### Background

Many methods in basic research, diagnostics, therapeutics and forensics are based on the analysis of nucleic acids. More recently, highly parallel processes such as microarrays and next generation sequencing (also known as "massive parallel sequencing") have been developed that allow analysis of thousands, millions and even beyond billions of different nucleic acid molecules simultaneously. For this purpose, nucleic acids need to be transformed into a common format to be processed in parallel as a nucleic acid library. The optimal library format is to supply unknown nucleic acids with common flanking sequences, so called adapters. Since many of the analysis processes require nucleic acid amplification steps such as PCR, the flanking sequences represent binding sites for at least two different specific primers. The classic method to add adapters to unknown nucleic acids is to first generate blunt-ended double-stranded DNA fragments. These fragments are then incubated with two different blunt-ended double-stranded adapters to be ligated with a ligase such as T4 DNA ligase. In order to avoid excess adapter dimers, only the blunt ended double-stranded DNA fragments are phosphorylated and only one end of the adapters can be ligated. However, the ligation efficiency using blunt end DNA is lower than sticky ends. In addition, due to permutations of fragments being ligated to two different adapters, only one half of the ligation products yield different adapters at both ends. Therefore, only one half of the ligation products can be efficiently amplified in PCR. Newer approaches are directed at using one adapter with two different primer binding sites that are ligated to both 5' and 3'-ends of each terminus of a given double-stranded DNA fragment. Thus, each of the two strands of a double-stranded DNA fragment is supplied with different primer binding sites for efficient PCR amplification. By adding a defined 3' single base overhang to the double-stranded DNA fragment and utilising adapters with a complementary single base overhang, the ligation reaction is much more efficient and does not yield any dimers or other multimers. Currently, two commercial approaches solve these problems associated with the classical adapters. One approach disclosed in WO 2007/052006 A1 is based on a Y-shaped adapter format which represents two annealed oligonucleotides comprising a complementary double-stranded region at the ligatable end and a forked, or tailed end of unpaired nucleotides comprising distinct sequences that can be used as specific primer binding sites. Another approach disclosed in WO 2009/133466 A2, is based on a hairpin-forming adapter comprising one partially annealed oligonucleotide which comprises a complementary double-stranded region at the ligatable 5' and 3' termini and a looping, connecting part comprising distinct sequences that can be used as specific primer binding sites. The loop comprises a scissile moiety which has to be cleaved after ligation in an additional processing step in order to be compatible with downstream nucleic acid library processing steps such as PCR. The hairpin and Y-adapter formats yield common sequences flanking both ends of the nucleic acid library insert, which requires longer primers in sequencing in order to render the sequencing reaction specific for only one of the ends. Another, yet non-commercial adapter format disclosed in WO 2009/133466 A2 is based on an adapter that yields different restriction enzyme cleavage sites downstream of the primer binding site after the first primer extension step. This allows ligation with a second adapter comprising a different primer binding site after cleavage. However, these methods comprise additional and cumbersome processing steps or require long adapter oligonucleotides which are less efficient in chemical synthesis and ligation compared to simple double-stranded adapters. There is a need to provide a method that does not require complicated enzymatic processing steps after ligation prior to PCR and utilises shorter, double-stranded adapter molecules without complex structures that do not yield common flanking sequences at the generated nucleic acid library.

### Summary of Invention

The present invention is defined by the appended claims.

The present invention solves the technical problem of providing a method based on a single adapter molecule without elongated oligonucleotides comprising forked or hairpin structures that is well accepted by ligating enzymes and does not require additional steps after ligation for generating a nucleic acid library without extensive common sequences at the termini.

A solution to the technical problem is providing an at least partially double-stranded adapter, which sequence is converted after ligation, said adapter comprising one or more modified bases in the region of a primer binding site that is ligated to both ends of a nucleic acid fragment and converted to at least two distinct primer binding sites for an efficient amplification reaction with more than one specific primer.

The method according to the invention has the advantage of reducing the length of adapter nucleotides to the primer binding sites. Alternatively, a substantially single-stranded adapter can be used of which only one strand needs to be ligated, which is advantageous in combination with ligating enzymes such as topoisomerases and transposases that can only join one strand. Adapter size reduction results in a more efficient and economic synthesis of the oligonucleotides and minimises common flanking sequences in a nucleic acid library to optional elements such as barcode and enzyme recognition sites. Therefore, a sequencing primer may not comprise 3' ends that could anneal to both ends of a template, which also reduces the requirement for stronger and specific binding of the 5' part of the primer. Ligating enzymes require defined double stranded nucleic acids for efficient ligation, which is achieved by omitting problematic structures such as single-stranded tails and loops. The whole library generation process may not require separate and intermediate enzymatic polishing steps after ligation as the sequence conversion by a polymerase represents an integral part of the mandatory amplification step.

A first aspect of the invention relates to a method of producing an asymmetrically tagged nucleic acid fragment, said method comprising the steps:
i. ligating an adapter to each end of a double-stranded nucleic acid fragment, wherein said adapter comprises a ligation site and a primer-binding region; and
ii. converting a first adapter sequence in the primer-binding region into a primer-binding site, particularly by direct conversion of at least one base comprised within the first adapter; or duplicating the first adapter sequence into the primer-binding site, wherein primer-binding site is complementary or identical to first adapter sequence except at least one position, and/or
iii. performing a step of nucleic acid synthesis initiated from a primer hybridised to a primer-binding site and generating a second primer-binding site, thereby producing an asymmetrically tagged nucleic acid fragment.

In certain embodiments, the primer-binding region of the adapter comprises one, preferably two or more base(s) that are directly converted in step ii) of the method of producing an asymmetrically tagged nucleic acid fragment to differently coding base(s).

In certain embodiments, the direct conversion of the base(s) in the primer binding region in the method of producing an asymmetrically tagged nucleic acid fragment is performed by an enzymatic, chemical or photochemical reaction.

In certain embodiments, the enzymatic direct conversion of bases in the method of producing an asymmetrically tagged nucleic acid fragment is performed by a nucleic acid repair enzyme, an acetyl esterase and/or penicillin G acylase.

In certain embodiments, the chemical direct conversion of bases in the method of producing an asymmetrically tagged nucleic acid fragment is performed by removal of a masking group by a Staudinger reaction.

In certain embodiments, the method of the invention of producing an asymmetrically tagged nucleic acid fragment comprises an adapter comprising one or more universal bases and/or convertible bases in the primer binding region that are not unmodified adenine, cytosine, guanine, thymidine, or uracil.

In certain embodiments, the universal base is select from inosine (hypoxanthine), xanthine, oxanine, 8-oxo-guanine, nebularine, 3-nitropyrrole, 5-nitroindole, 4-methylindole, O4-methyl-thymidine (O4mT), O4-ethyl-thymidine (O4eT), 6H,8H-3,4-dihydro-pyrimido[4,5-c][1,2]oxazin-7-one (P), N6-methoxy-2,6-diaminopurine (K), a 5-fluoroindole base, pyrrolidine, a dSpacer (1',2'-dideoxyribose) or an abasic site.

In another embodiment, the method the invention of producing an asymmetrically tagged nucleic acid fragment comprises an adapter comprising one or more universal bases and/or convertible bases in the primer binding region, wherein particularly one of more bases comprised within the adapter, particularly one ore more universal bases and/or convertible bases, are modified bases.

In certain embodiments, the convertible base comprises O4-methyl-thymidine, O6-methyl-guanine, S4-methyl-thio-uridine, S4-methyl-thio-thymidine, S6-methyl-thio-guanine, N1-methyl-adenine, N3-methyl-adenine, C8-methyl-guanine, N2,3-etheno-guanine, O6-methyl-hypoxanthine, O4-ethyl-thymidine, O6-ethyl-guanine, S4-ethyl-thio-uridine, S4-ethyl-thio-thymidine, S6-ethyl-thio-guanine, N1-ethyl-adenine, N3-ethyl-adenine, C8-ethyl-guanine, O6-ethyl-hypoxanthine, S4-thio-uridine, S4-thiothymidine, S6-thio-guanine, 1-ethynyl-dSpacer (abasic site with an alkyne group), or a 1-thiol-dSpacer (abasic site with a thiol group).

In certain embodiments, the first adapter sequence comprising one or more universal bases and/or convertible bases in the primer binding region is converted by a first polymerase with a first base bias in step ii) of the method of producing an asymmetrically tagged nucleic acid fragment; and wherein a primer is specifically hybridised to the primer binding site converted by a second polymerase with a second base bias in step iii).

In certain embodiments, the primer binding region of the adapter of producing an asymmetrically tagged nucleic acid fragment is converted by at least two polymerases with different base incorporation bias, wherein the different bias preferably obeys the "A" rule or "C" rule.

In certain embodiments, the adapter primer-binding region in the method of producing an asymmetrically tagged nucleic acid fragment comprises one or more universal and/or convertible bases is double-stranded and both strands are ligated to both ends of the nucleic acid fragment.

In certain embodiments, the one or more universal base in the method of producing an asymmetrically tagged nucleic acid fragment is converted by specific primer annealing thereto and subsequent polymerase extension.

In certain embodiments, the method of producing an asymmetrically tagged nucleic acid fragment comprises the steps of:
i. ligating a first adapter nucleic acid molecule to a template strand of a double-stranded nucleic acid fragment and a second adapter nucleic acid molecule to a complementary strand of the double-stranded nucleic acid fragment, wherein the first and said second adapter nucleic molecule acid are characterized by an identical base sequence and comprise a ligation site and a primer-binding region, and the primer-binding region comprises at least one universal base and/or at least one convertible base, and wherein optionally the first adapter nucleic acid molecule and the second adapter nucleic acid molecule are at least partially double-stranded, and wherein optionally both strands are of the first and said second adapter nucleic acid molecule are ligated to the double-stranded nucleic acid fragment,
ii. optionally converting the at least one convertible base comprised with the first and/or the second adapter nucleic acid molecule into an at least one converted base;
iii. obtaining a first antisense adapter nucleic molecule and a second antisense adaptor nucleic acid molecule by performing a first extension step, wherein
   - the complementary strand is extended yielding said first antisense adapter nucleic acid molecule ligated to the complementary strand, and the template strand is extended yielding a second antisense adapter nucleic acid molecule ligated to the template strand, or
   - a primer is annealed to a strand of the at least partially double stranded second adapter nucleic acid molecule ligated to the template strand and extended yielding a nucleic acid strand comprising said primer, a nucleic acid sequence complementary to said template strand, and said first antisense adapter nucleic acid molecule, and
   - a primer is annealed to a strand of the at least partially double stranded first adapter nucleic acid molecule ligated to the complementary strand and extended yielding a nucleic acid strand comprising the primer, a nucleic acid sequence complementary to the complementary strand, and the second antisense adapter nucleic acid molecule,
wherein the first antisense adapter nucleic acid molecule is essentially complementary to the first adapter nucleic acid molecule, and the second antisense adapter nucleic acid molecule is essentially complementary to the second adapter nucleic acid except at positions opposite to the at least one universal, convertible or converted base comprised within the first and second adapter nucleic acid molecule, thereby yielding an asymmetrically tagged nucleic acid fragment.

In certain embodiments, the at least one convertible base and the at least one converted base preferably pair with different bases.

In certain embodiments, the conversion of the at least one convertible base comprised within the primer binding region of the first and/or said second adapter nucleic acid molecule is performed by an enzymatic, chemical or photochemical reaction, particularly by a nucleic acid repair enzyme, an acetyl esterase and/or penicillin G acylase.

In certain embodiments, the method of the invention comprises
- the first extension step, wherein the complementary strand of the double-stranded nucleic acid fragment is extended yielding the first antisense adapter nucleic acid molecule ligated to the complementary strand, and the template strand double-stranded nucleic acid fragment is extended yielding the second antisense adapter nucleic acid molecule ligated to the template strand, and
- a second extension step, wherein
   - a primer is annealed to the second antisense nucleic acid molecule and extended yielding a nucleic acid strand comprising the primer, a nucleic acid complementary to the complementary strand, and a third antisense adapter nucleic acid molecule,
   - a primer is annealed to the first antisense adapter nucleic acid molecule, and extended yielding a nucleic acid strand comprising said primer, a nucleic acid complementary to the template strand, and a fourth antisense adapter nucleic acid molecule, and
   - the third antisense adapter nucleic acid molecule is essentially complementary to the first adapter nucleic acid molecule and the second antisense adapter nucleic acid molecule is essentially complementary the second adapter nucleic acid except at positions opposite to the at least one universal, convertible or converted base comprised within the first and second adapter nucleic acid molecule, and the third and fourth antisense adapter nucleic acid molecule are characterized by different base sequences, thereby yielding an asymmetrically tagged nucleic acid fragment.

In certain embodiments, the method of the invention comprises
a) the first extension step, wherein
   - a primer is annealed to the strand of the at least partially double-stranded second adapter nucleic acid molecule ligated to the template strand and extended yielding the nucleic acid strand comprising the primer, the nucleic acid complementary to the complementary strand, and the first antisense adapter nucleic acid molecule and, and
   - a primer is annealed to the strand of the at least partially double-stranded first adapter nucleic acid molecule ligated to the complementary strand and extended yielding the nucleic acid strand comprising the primer, the nucleic acid complementary to the template strand, and the second antisense adapter nucleic acid molecule and, and
b) a second extension step, wherein
   - a primer is annealed to the second antisense nucleic acid molecule and extended yielding a nucleic acid strand comprising the primer, a nucleic acid complementary to the complementary strand, and a third antisense adapter nucleic acid molecule, and
   - a primer is annealed to the first antisense adapter nucleic acid molecule, and said primer is extended yielding a nucleic acid strand comprising the primer, a nucleic acid complementary to the template strand, and a fourth antisense adapter nucleic acid molecule, and
   - the third antisense adapter nucleic acid molecule is essentially complementary to the
first adapter nucleic acid molecule and the fourth antisense adapter nucleic acid molecule is essentially complementary the second adapter nucleic acid except at positions opposite to said universal, convertible or converted bases comprised within the first and second adapter nucleic acid molecule, and the third and fourth antisense adapter nucleic acid molecule are characterized by different base sequences, thereby yielding an asymmetrically tagged nucleic acid fragment.

In certain embodiments, the first extension step is performed by a first polymerase with a first base bias, and the second extension steps is by a second polymerase with a second base bias, e.g. the first polymerase follows the A-rule and the second polymerase follow the C-rule or vice versa.

In certain embodiments, a primer anneals to the strand of the at least partially double stranded second adapter nucleic acid molecule ligated to the template strand and to the first and/or third antisense adapter nucleic acid molecule at different temperatures, particularly differing by at least 2°C, 5°C or 10°C.

In certain embodiments, a primer anneals to the strand of said at least partially double stranded first adapter nucleic acid molecule ligated to the complementary strand and to the second and/or fourth antisense adapter nucleic acid molecule at different temperatures, particularly differing by at least 2°C, 5°C or 10°C.

In certain embodiments, the adapter ligation to the nucleic acid fragment in step i) of the method of producing an asymmetrically tagged nucleic acid fragment is performed by a ligase, a topoisomerase, a recombinase or a transposase.

In certain embodiments, the adapter, particularly the first and/or second adapter nucleic acid molecule, of the method of producing an asymmetrically tagged nucleic acid fragment comprises one or more of the following: a barcode, a recombination site, a topoisomerase recognition site, or a transposase recognition site.

In certain embodiments, the sequences in the method of the invention of producing an asymmetrically tagged nucleic acid fragment the converted primer-binding sites have lower annealing temperatures with the respective non-cognate primer than at least 2°C, preferably more than 5°C and most preferably more than 10°C.

In certain embodiments, the first extension step for converting the first adapter sequence into a primer binding site to generate an asymmetrically tagged nucleic acid fragment comprises annealing conditions that differ from later amplification steps, wherein preferably the temperature is lower in said first extension step.

In certain embodiments, the method of the invention of producing an asymmetrically tagged nucleic acid fragment uses primers with 3' blocking ends that can be unblocked upon specific hybridisation to the cognate primer-binding site, said unblocking preferably performed by a nucleic acid repair enzyme. In certain embodiments, the method of the invention of producing an asymmetrically tagged nucleic acid fragment uses RNAseH or Endonuclease IV for highly specific 3' unblocking of annealed primers. In certain embodiments, the method of the invention of producing an asymmetrically tagged nucleic acid fragment comprises the use of a polymerase for extension and/or amplification selected from the group consisting of: a RNA polymerase, a mesophilic DNA polymerase, a reverse transcriptase, a repair polymerase (family X), and a thermophilic DNA polymerase capable of copying a universal base in a second nucleic acid strand of the adapter.

In certain embodiments, the method of the invention of producing an asymmetrically tagged nucleic acid fragment comprises synthesis steps performed on a solid phase, preferably a bridge amplification using immobilised primers.

In certain embodiments, the method of the invention of producing an asymmetrically tagged nucleic acid fragment is used for generating nucleic acid libraries suitable for massive parallel sequencing.

In a second aspect, the disclosure relates to an at least partially double-stranded adapter comprising:
a) one or more universal and/or convertible base in a primer binding region, wherein the sequences of the converted primer-binding sites have a lower melting temperature when hybridised to each other than with the cognate primer; and
b) a ligation site positioned on a first end of said adapter configured to allow ligation of said adapter to a compatible end of a double stranded nucleic acid fragment.

In certain embodiments, the one or more universal and/or convertible base of the at least partially double-stranded adapter base is a modified base and not an unmodified base such as adenine, cytosine, guanine, thymidine or uracil.

In certain embodiments, the at least partially double-stranded adapter nucleic acid molecule comprises:
a) a primer binding region comprising one or more universal and/or convertible base, and
b) a ligation site positioned on a first end of said adapter nucleic acid molecule configured to allow ligation of said adapter to a compatible end of a double stranded nucleic acid fragment,
wherein said primer binding region is convertible or copyable into an alternative primer binding region, and said primer binding region and said alternative primer binding region can be hybridized with the same primer at different temperatures.

In certain embodiments, the universal base is select from inosine (hypoxanthine), xanthine, oxanine, 8-oxo-guanine, nebularine, 3-nitropyrrole, 5-nitroindole, 4-methylindole, O4-methyl-thymidine (O4mT), O4-ethyl-thymidine (O4eT), 6H,8H-3,4-dihydro-pyrimido[4,5-c][1,2]oxazin-7-one (P), N6-methoxy-2,6-diaminopurine (K), a 5-fluoroindole base, pyrrolidine, a dSpacer (1',2'-dideoxyribose) or an abasic site.

In certain embodiments, the convertible base comprises O4-methyl-thymidine, O6-methyl-guanine, S4-methyl-thio-uridine, S4-methyl-thio-thymidine, S6-methyl-thio-guanine, N1-methyl-adenine, N3-methyl-adenine, C8-methyl-guanine, N2,3-etheno-guanine, O6-methyl-hypoxanthine, O4-ethyl-thymidine, O6-ethyl-guanine, S4-ethyl-thio-uridine, S4-ethyl-thio-thymidine, S6-ethyl-thio-guanine, N1-ethyl-adenine, N3-ethyl-adenine, C8-ethyl-guanine, O6-ethyl-hypoxanthine, S4-thio-uridine, S4-thiothymidine, S6-thio-guanine, 1-ethynyl-dSpacer (abasic site with an alkyne group), or a 1-thiol-dSpacer (abasic site with a thiol group).

In certain embodiments, the at least partially double-stranded adapter, particularly the at least partially double-stranded adapter nucleic acid molecule, comprises a blocking modification between primer-binding regions in one strand.

In certain embodiments, the at least partially double-stranded adapter, particularly the at least partially double-stranded adapter nucleic acid molecule, comprises one or more backbone and/or base modifications that increase the melting temperature at the primer binding site.

In certain embodiments, the at least partially double-stranded adapter, particularly the at least partially double-stranded adapter nucleic acid molecule, comprises one or more of the following: a barcode, a restriction enzyme site, a recombination site, topoisomerase recognition site.

In certain embodiments, the at least partially double-stranded adapter, particularly the at least partially double-stranded adapter nucleic acid molecule, comprises a sequence which can be converted to one of the sequences of SEQ-ID NO:1-11 according to the method of producing an asymmetrically tagged nucleic acid fragment with a homology of less than 2, preferably 1 and most preferably no base exchanges.

In a third aspect, the disclosure relates to the use of an at least partially double-stranded adapter nucleic acid molecule, particularly an at least partially double-stranded adapter nucleic acid molecule according to the above aspect or embodiments of the disclosure, in an amplification reaction is provided, wherein the at least partially double-stranded adapter nucleic acid molecule particularly comprises one or more universal and/or convertible base in a primer binding region, wherein the sequences of the converted primer-binding sites have a lower melting temperature when hybridised to each other than with the cognate primer; and a ligation site positioned on a first end of said adapter configured to allow ligation of said adapter to a compatible end of a double stranded nucleic acid fragment in an amplification reaction of a nucleic acid.

In a fourth aspect, the disclosure relates to a kit for use in preparing a library of asymmetrically tagged nucleic acid molecules comprising:
a) a convertible adapter according to the method of the disclosure, particularly an at least partially double-stranded adapter nucleic acid molecule according to the above aspect or to any one of the above embodiments of the disclosure;
b) one or more primers capable of annealing to the primer-binding sites of the adapter after conversion; and
c) one or more of the following ligating enzymes: a ligase, a topoisomerase, a transposase, a recombinase.

In certain embodiments, the kit may also comprise at least one converting reagent, wherein the converting reagent is an enzyme, chemical or photochemical reagent.

In certain embodiments, the kit may also comprise at least one converting reagent, wherein the converting enzyme is a nucleic acid repair enzyme, an acetyl esterase and/or penicillin G acylase.

In certain embodiments, the kit may also comprise at least one converting reagent, wherein the converting nucleic acid repair enzyme is an alkylation damage repair enzyme, preferably selected from one of the following: AlkA, AlkB, Ada, Aid and Ogt.

In certain embodiments, the kit may also comprise at least one polymerase with a bias for cytosine incorporation opposite of universal bases, preferably opposite of an abasic site or hydrophobic stacking bases.

In certain embodiments, the kit may also comprise at least one blocked primer and at least one unblocking enzyme, wherein said unblocking enzyme is preferably an RNAseH or endonucleaseIV. In a fifth aspect, the invention relates to a method of producing an asymmetrically tagged nucleic acid fragment for bisulfite sequencing comprising the following steps:
i. ligating an adapter to each end of a double-stranded nucleic acid fragment, wherein said adapter comprises a ligation site and a primer-binding region and at least two non-methylated cytosine bases;
ii. converting a first adapter sequence in the primer-binding region into a primer-binding site by bisulfite treatment; and
iii. performing a step of nucleic acid synthesis initiated from a primer hybridised to a primer-binding site and generating a second primer-binding site, thereby producing an asymmetrically tagged nucleic acid fragment suitable for bisulfite sequencing.

### Brief Description of Drawings

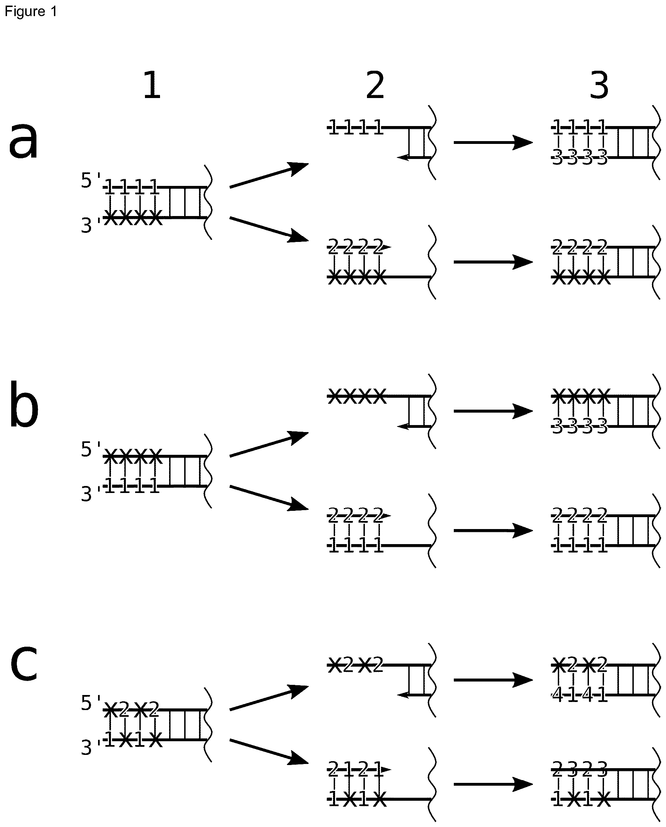

**Figure 1****:** Base conversion mechanisms depending on adapter strand orientation. The adapter is depicted as a double-stranded nucleic acid ligated to an unknown fragment. The 5' and 3' ends of the strands are indicated in the first step and most of the ligated fragment is masked for simplicity. The universal and/or convertible bases are depicted as X, while defined bases are indicated by a number. The adapter may comprise only one X, or other defined nucleotides between the X. The fate of both ligated adapter strands in a first conversion step are shown. a) discloses a base conversion, wherein the universal and/or convertible bases in the primer-binding site of the ligated adapter in step 1 are converted by primer annealing in step 2 and fixed by polymerase extension in step 3. b) discloses a base conversion by polymerase, wherein the X in the primer-binding region are converted by copying process of the polymerase alone. c) discloses the combination of both conversion mechanisms, wherein X is present in both adapter strands.

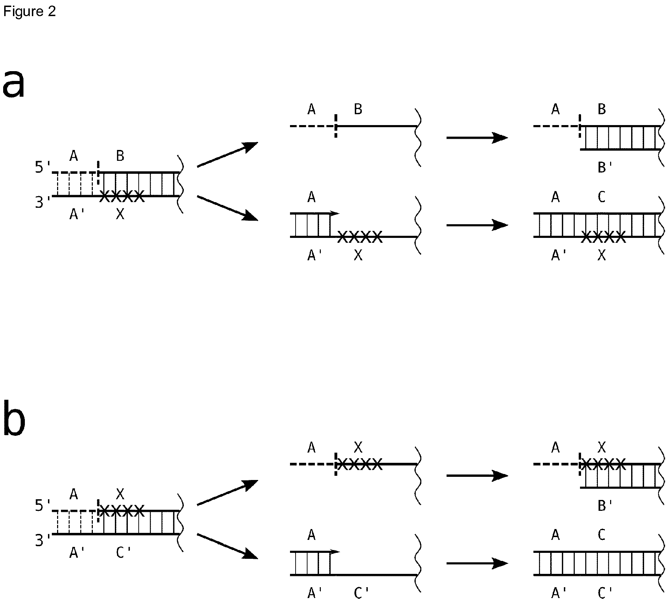

**Figure 2****:** Base conversion in adapters with non-overlapping primer-binding sites. The adapter is depicted as a double-stranded nucleic acid ligated to an unknown fragment. The 5' and 3' ends of the strands are indicated in the first step and most of the ligated fragment is masked for simplicity. The universal and/or convertible bases are depicted as X, while defined bases are indicated by a number. The adapter may comprise only one X, or other defined nucleotides between the X. Primer binding sites are indicated by the letters A, B, and C, and their respective antisense A', B' and C'. The optional A part and the blocking site in the upper strand of the adapter are indicated by broken lines. The fate of both ligated adapter strands in a first conversion step are shown. a) discloses the conversion of X in the primer-binding-region opposite to B. b) discloses the conversion of X in the strand opposite to the primer-binding site C'. The converted products can be efficiently amplified by primer sets comprising the sequences of A and B or B and C.

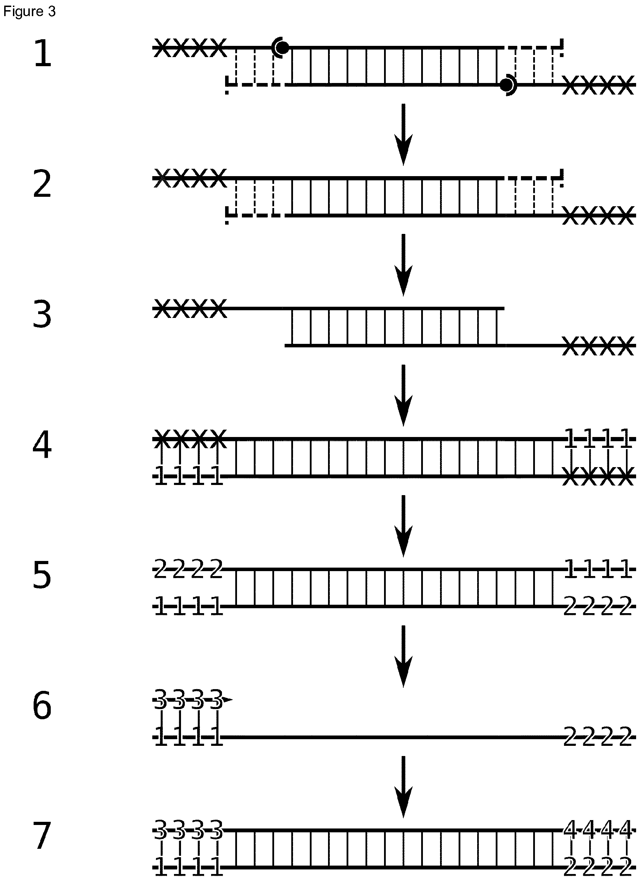

**Figure 3****:** Method for adapter conversion with a single-stranded primer-binding region comprising directly convertible bases. Directly convertible bases are depicted as X, while defined bases are indicated by a number. The adapter may comprise only one X, or other defined nucleotides between the X. The ligatable 3' end of the primer binding region comprising strand of the adapter is represented as a semi circle and the compatible end at the 5' ends of the double stranded nucleic acid fragment. Optional double stranded region of the adapter and the blocked 3' end are depicted as broken lines. In step 1, both adapter and double-stranded nucleic acid fragment are provided and ligated to both ends of the fragment in step 2. The optional double stranded region of the adapter can be removed in an optional step 3. The 3' end of the ligated nucleic acid fragment is elongated by a polymerase in step 3, leading to a first sequence 1 opposite of the convertible base(s) in step 4. The convertible base(s) are converted to a base 2 not matching base 1 in the opposite strand. A primer comprising a base 3 specific for base 1 can hybridise to the new primer-binding site in step 6 and elongated to copy base 2 to a matching base 4, thus resulting in an asymmetrically tagged nucleic acid fragment in step 7.

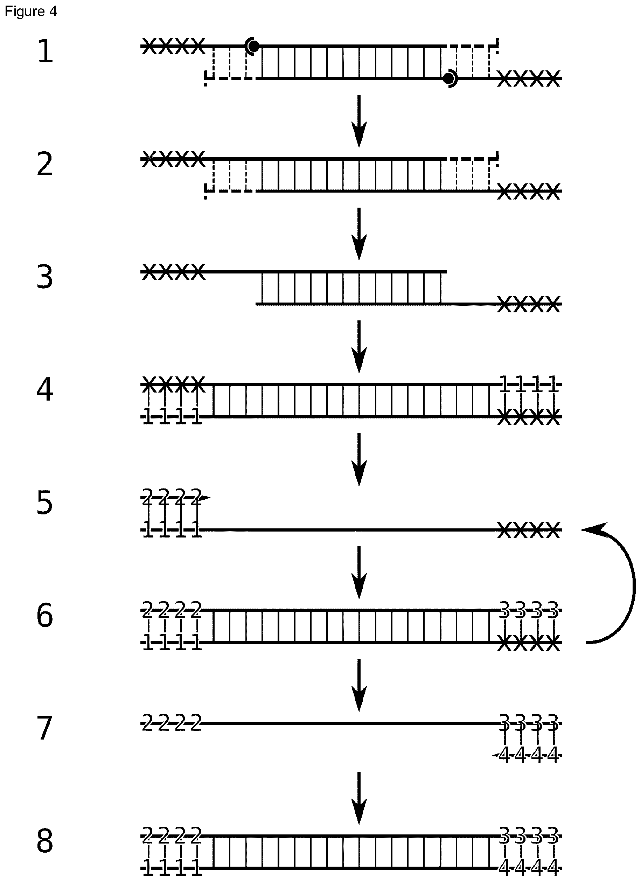

**Figure 4****:** Method for adapter conversion with a single-stranded primer-binding region comprising universal bases using polymerases with different conversion bias. Universal bases are depicted as X, while defined bases are indicated by a number. The adapter may comprise only one X, or other defined nucleotides between the X. The ligatable 3' end of the primer-binding region comprising strand of the adapter is represented as a semi circle and the compatible end at the 5' ends of the double stranded nucleic acid fragment. Optional double-stranded region of the adapter and the blocked 3' end are depicted as broken lines. In step 1, both adapter and double-stranded nucleic acid fragment are provided and ligated to both ends of the fragment in step 2. The optional double stranded region of the adapter can be removed in an optional step 3. The 3' end of the ligated nucleic acid fragment is elongated by a polymerase with bias for a base 1 in step 4. A primer comprising a base 2 specific for base 1 can hybridise to the new primer-binding site in step 5 and is elongated by a polymerase with bias for base 3, thus generating a new primer binding site in step 6. Denaturation after step 6 recycles the strand comprising the universal bases to step 5. The other strand is bound by a primer comprising a base 4 which is specific for base 3 in the primer-binding site in step 7 and elongated to from an asymmetrically tagged nucleic acid fragment in step 8.

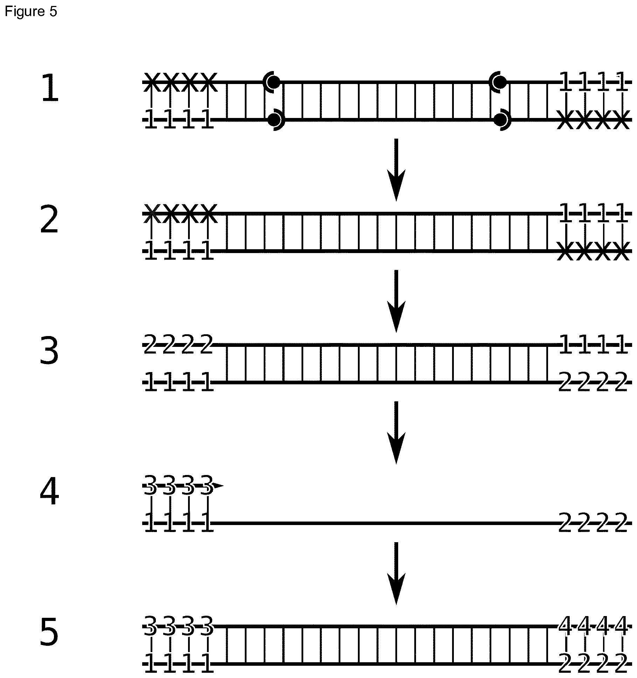

**Figure 5****:** Method for adapter conversion with a double-stranded primer-binding region comprising directly convertible bases. Directly convertible bases are depicted as X, while defined bases are indicated by a number. The adapter may comprise only one X, or other defined nucleotides between the X, wherein the other strand comprises a matching base 1 opposite of X. The ligatable 3' end of the primer-binding region comprising strand of the adapter is represented as a semi circle and the compatible end at the 5' ends of the double-stranded nucleic acid fragment. The 5' end of the other adapter strand and the 3' end of the fragment also comprise ligatable groups. In the first step both strands are ligated to form a substantially continuous double-stranded fragment with adapters on both ends in step 2. The convertible base X is converted to base 2 in step 3 that forms a mismatch with base 1. A primer comprising a base 3 which is specific for base 1 in the primer-binding site is hybridised in step 4 and elongated in step 5 to from an asymmetrically tagged nucleic acid fragment.

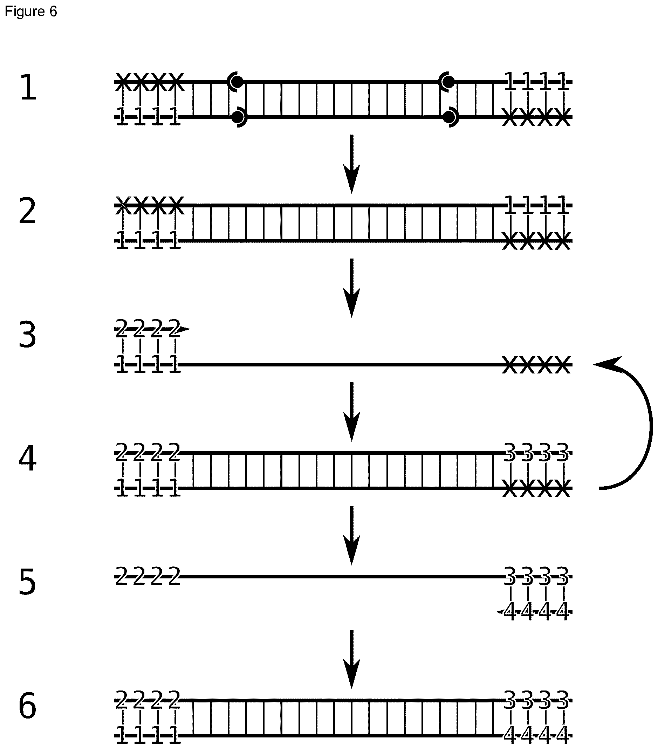

**Figure 6****:** Method for adapter conversion with a double-stranded primer-binding region comprising universal bases. Universal bases are depicted as X, while defined bases are indicated by a number. The adapter may comprise only one X, or other defined nucleotides between the X, wherein the other strand comprises a matching base 1 opposite of X. The ligatable 3' end of the primer-binding region comprising strand of the adapter is represented as a semi circle and the compatible end at the 5' ends of the double stranded nucleic acid fragment. The 5' end of the other adapter strand and the 3' end of the fragment also comprise ligatable groups. In the first step both strands are ligated to form a substantially continuous double-stranded fragment with adapters on both ends in step 2. A primer comprising a base 2 specifically binding to base 1 is hybridised to the first primer-binding site in step 3 and elongated by a polymerase converting X to base 3 in the new strand, thus forming a new specific primer-binding site in step 4. Denaturation after step 4 recycles the strand comprising the universal bases to step 3. The other strand is bound by a primer comprising a base 4 which is specific for base 3 in the primer-binding site in step 5 and elongated to from an asymmetrically tagged nucleic acid fragment in step 6.

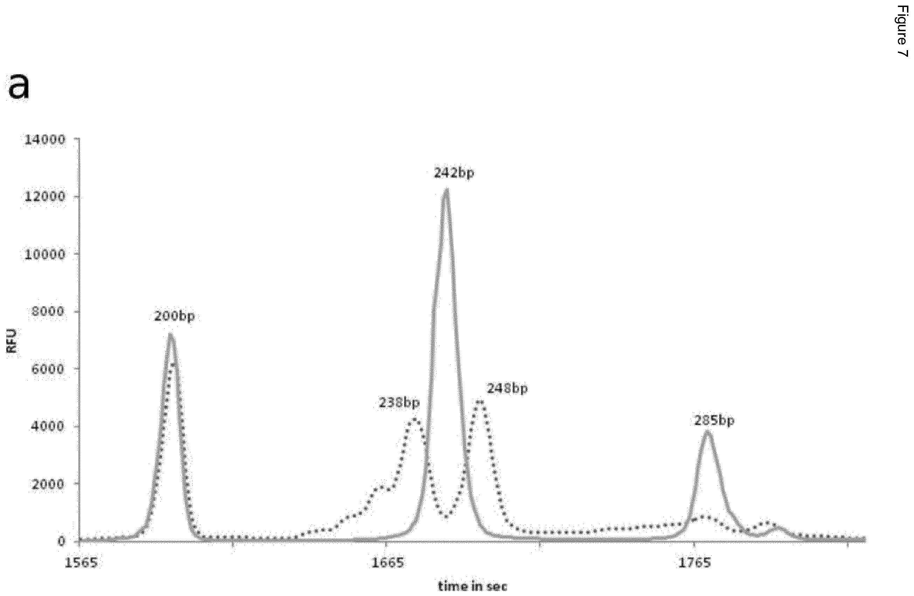

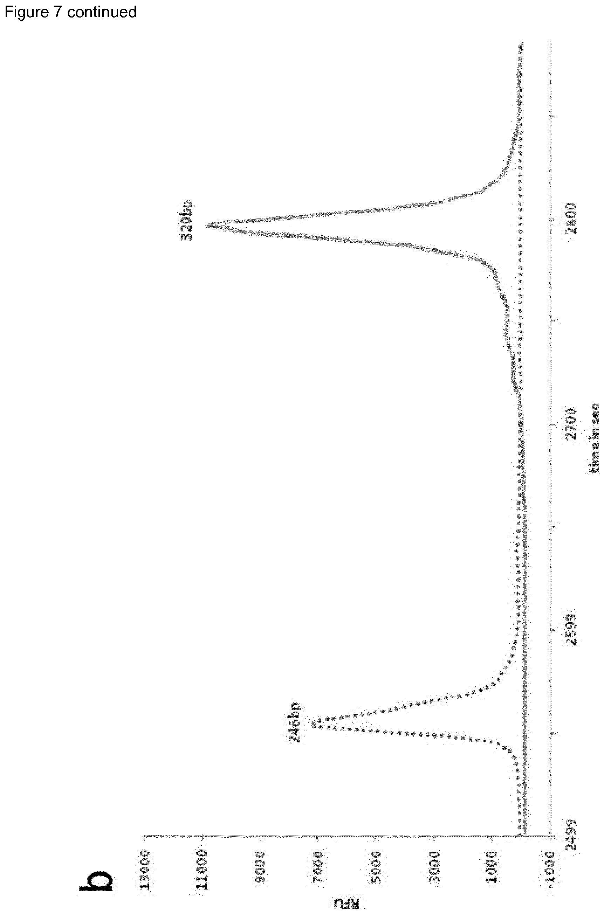

**Figure 7****:** Electropherograms for direct comparison of ligation efficiencies of conventional adapters and convertible adapter according to the disclosure. The calculated main fragment size is depicted on top of each peak. The continuous lines represent results obtained for the conventional adapter sample and the dashed lines represents results obtained for the convertible adapter sample. a) discloses the products obtained directly after ligation without any amplification. The peak at 200 bp represents the unligated template, peaks between 238 bp and 248 bp represent DNA template ligated with adapter at one end, whereas peaks in the range of 285 bp represent complete ligation products with adapters at both ends. b) discloses the products that could be functionally amplified for 14 PCR cycles after ligation. The correct size of PCR product is found at 246 bp for conventional adapters and at 320 bp for the convertible adapter.

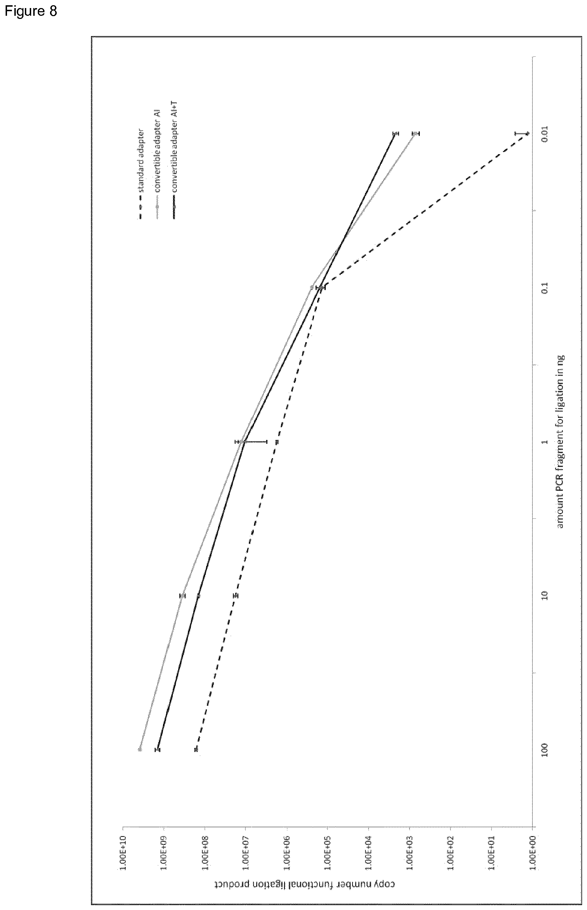

**Figure 8****:** Double logarithmic representation of adapter ligation efficiency at different fragment concentrations determined by digital droplet PCR (ddPCR). Data corresponding to convertible adapter with T-overhang is drawn as solid black line, convertible adapter as solid grey line, and the standard adapter as a dashed line. Error bars denote standard deviation of triplicate experiments.

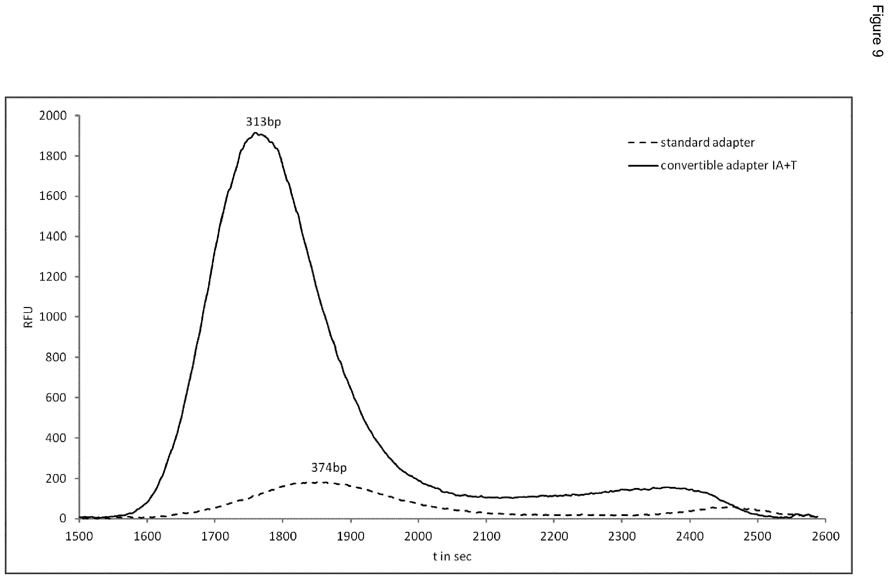

**Figure 9****:** Electropherogram for direct comparison of ligation efficiencies of conventional adapters and convertible adapter according to the disclosure after sizing and PCR. The convertible adapter with T overhang (AI+T) is drawn as a solid line and the standard adapter as a dashed line. The main peak is labelled with the calculated predominant fragment size.

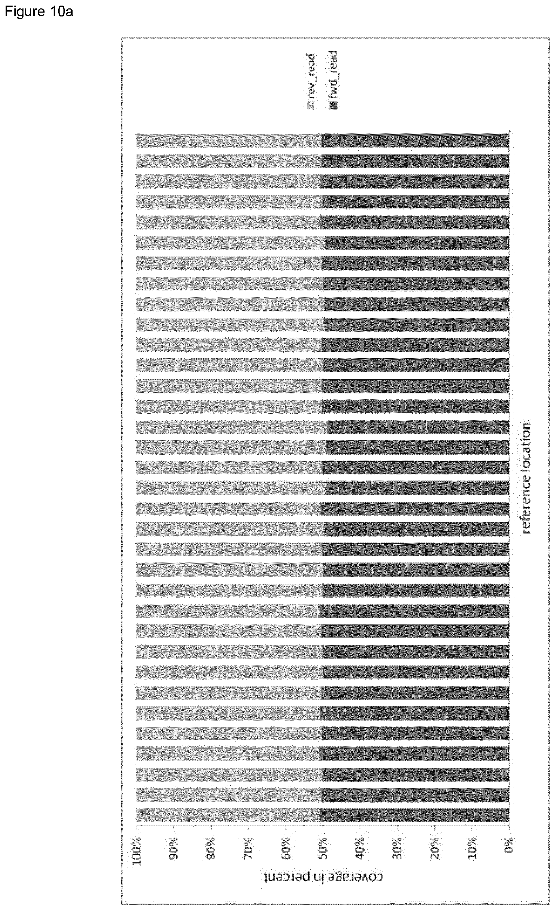

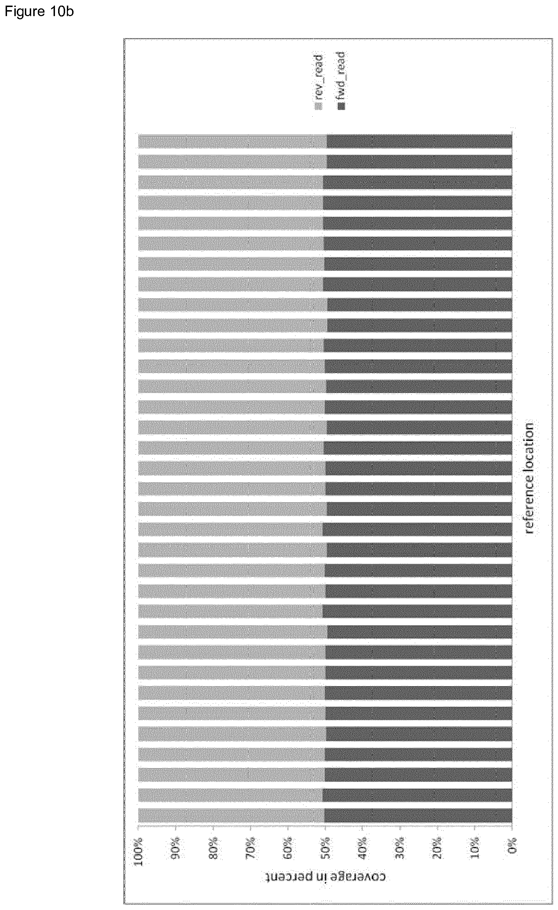

**Figure 10****:** Representation of mapped read distribution of genomic E. coli libraries against the reference genome. Both reverse and forward orientation is shown to indicate any bias due to library generation. A) represents the mapped sequences of the convertible IA+T library and B) represents the sequences of the standard adapter library.

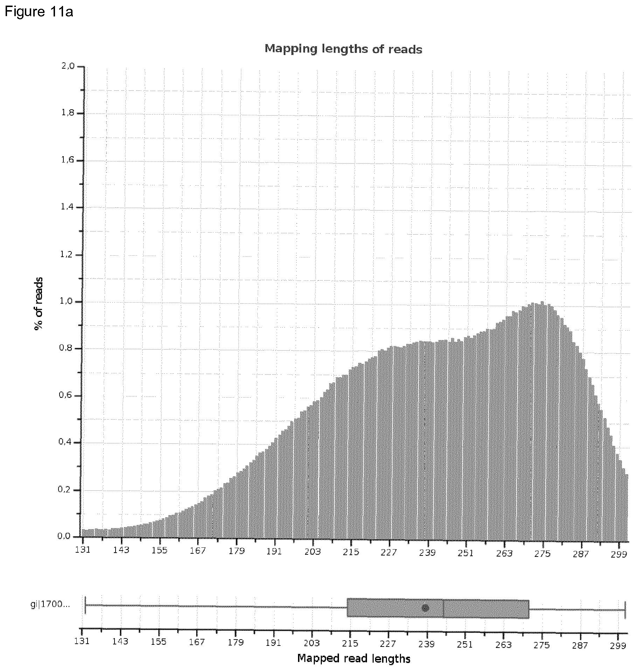

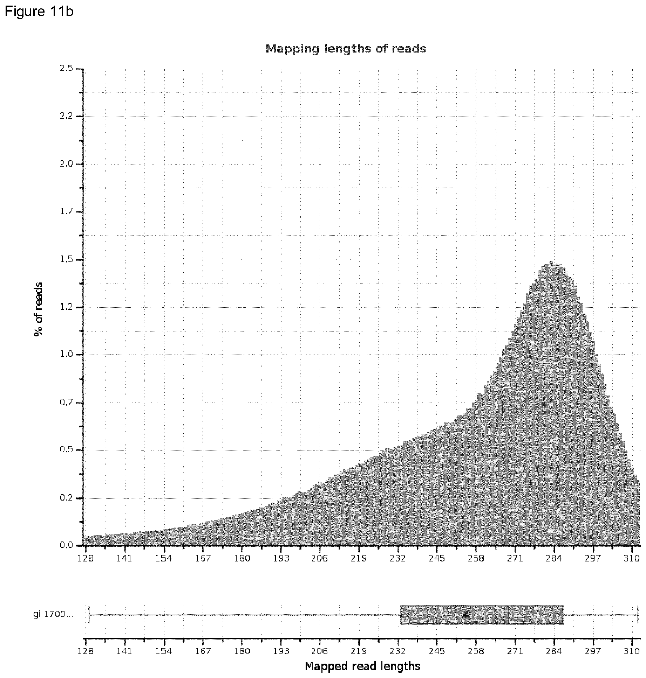

**Figure 11****:** Size distribution of mapped reads. A) represents the sizes of mapped reads of the convertible IA+T library and B) represents the sizes for the standard adapter library.

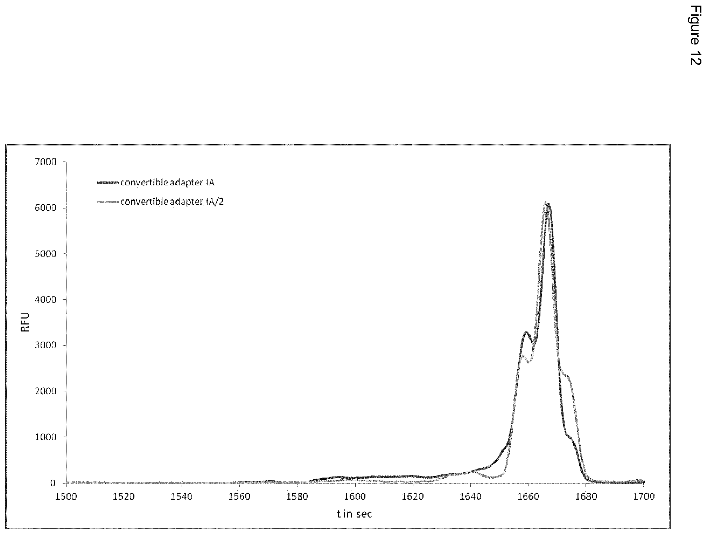

**Figure 12****:** Electropherogram for direct comparison of ligation and PCR efficiency of convertible adapter IA and convertible adapter IA/2. The black line corresponds to convertible adapter IA and the grey line corresponds to convertible adapter IA/2.

### Definitions

### Ligase

The term "ligase" used in its broadest sense refers to an enzyme belonging to the category EC: 6.5 (Enzyme Commission number 6.5) that is able to join ends of nucleic acids. Ligases may utilise nucleotide triphosphates, nicotinamide adenine dinucleotide (NAD), ADP-ribosylated 5'-ends, or cyclic 2',3' phosphates at 3' ends for joining the ends of nucleic acids. Also truncated or otherwise mutated variants of ligases such as that are only able to ligate pre-activated ends such as 5'-adenylated termini are within the scope of the invention. Preferred are ligases that are able to ligate sticky ends or single base overhangs. Particularly preferred is the DNA ligase of bacteriophage T4.

### Topoisomerase

The term "topoisomerase" refers to an enzyme belonging to the categories EC: 5.99.1.2 and 5.99.1.3. Preferably, the term refers to a type I topoisomerase (EC: 5.99.1.2). More preferably, the topoisomerase used for ligating an adapter to a nucleic acid fragment is a type IB topoisomerase. Most preferably, the enzyme is related to the vaccinia virus topoisomerase and recognises a 5'-(C/T)CCTT↓ target site for a site-specific cleavage. A suicide substrate bearing a terminal target site in double-stranded form with a single-stranded 3' extension can be cleaved by the topoisomerase resulting in a covalent enzyme adduct which can be transferred to 5' OH-groups of double-stranded nucleic acid fragments.

### Polymerase

The term "polymerase" used in its broadest sense refers to an enzyme belonging to the category EC: 2.7.7 (Enzyme Commission number 2.7.7) that is able to catalyse an extension of the 3'-end of a nucleic acid strand by one nucleotide at a time. Preferred are DNA-directed RNA polymerases (EC: 2.7.7.6), DNA nucleotidylexotransferases (EC: 2.7.7.31) and RNA-directed RNA polymerases (EC: 2.7.7.48). More preferred are DNA-directed DNA polymerase (EC: 2.7.7.7) and RNA-directed DNA polymerases (EC: 2.7.7.49). Most preferred are polymerases that are able to maintain their activity at elevated temperature such as Thermus aquaticus (Taq) DNA polymerase, or Thermus thermophilis (Tth) DNA polymerase, Pyrococcus furiosus (Pfu) DNA polymerase, Thermococcus sp. (9°N) DNA polymerase, Thermococcus gorgonarius (Tgo) DNA polymerase, Thermococcus litoralis DNA polymerase (Vent), truncations and domain fusions thereof. Especially preferred are mutant enzymes thereof which are able to copy universal bases like inosine, 8-oxo-guanine and/or can efficiently read through lesions such as abasic sites and may display different bias for base incorporation such as the "C" rule. In addition, natural polymerases that have translesion synthesis activity such as Family Y polymerases or primases are preferred polymerases to be used in combination with more processive enzymes. It is preferred that polymerases involved in the copying step of a universal base lack a 3'-exonuclease and/or lyase activity.

### Nucleic acid

The Term "nucleic acid" refers to a polymer of nucleotides. The polymer may include natural nucleosides (i.e., adenosine, thymidine, guanosine, cytidine, uridine, deoxyadenosine, deoxythymidine, deoxyguanosine, and deoxycytidine), nucleoside analogs (e.g. 2-aminopurine, 2-aminoadenosine, 2-thiothymidine, inosine (hypoxanthine), pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 5-hydroxybutynyl-2'-deoxyuridine, 6-thioguanine, S6-methyl-thioguanine, 7-deazaadenosine, 7-deazaguanosine, 8-oxoadenosine, 8-oxoguanosine, 06-methylguanine, and 2-thiocytidine), chemically modified bases, biologically modified bases (e.g., methylated bases), intercalated bases, abasic sites, ribose sugars (RNA), 2'-deoxyribose sugars (DNA), terminal 3'-deoxyribose or 2',3'-dideoxyribose sugars, modified sugars (e.g., 2'-fluororibose, arabinose, and hexose), or modified phosphate groups (e.g., phosphorothioates and 5'-N-phosphoramidite linkages). Furthermore, the backbone may include modified locked (LNA), unlocked (UNA), bridged (BNA), glycine (GNA) sugars, triazole-sugar (made by click chemistry), or a peptide backbone (PNA) or a mixture thereof.

### Oligonucleotide

The term "oligonucleotide" refers to a nucleic acid with a length of 4 to 100 nucleotides. Oligonucleotide may be modified to include labels for detection or identification such as fluorescent dyes or radioactive isotopes, haptens for capture, detection, or immobilisation such as biotin or digoxigenin, or reactive groups such as hydroxyl, phosphate, sulfonate ester, thiol, alkyne, azide, or EDC, for immobilisation, crosslinking, or derivatisation.

### Primer

The term "primer" herein may denote target-specific primer, each set comprising a forward and a reverse primer, random primers, degenerate primers, or random exonuclease-resistant primers depending on the number of nucleic acids to be amplified and the amplification method used. Primers can specifically anneal to their complementary target sequence under physiological buffer conditions. A free 3'-end may serve as a starting point for polymerases to elongate the primer strand using nucleotides as building blocks. However, primers may also comprise a blocked 3'-end which can be unblocked upon hybridisation for highly specific priming of target sequences. Primers herein also refer to low melting point primers, which usually have a length between 4 and 10 nucleotides with on average 6 nucleotides in length. The term "primer" also comprises a reaction mixture of nucleotides and a primase, which may be used to synthesize primers. Preferred primers bind specifically to their cognate targets at temperatures higher than 30°C and have a length of about 10 - 100, more preferably about 15 - 50, most preferably about 18 - 40 bases. Primers may be prepared using any suitable method, such as, for example, the phosphotriester and phosphodiester methods or automated embodiments thereof. In one such automated embodiment diethylophosphoramidites are used as starting materials. It is also possible to use a primer which has been isolated from a biological source (such as a restriction endonuclease digest).

### Primer-binding site

The term "primer-binding site" refers to a site within a nucleic acid strand to which a primer can specifically hybridise under stringent conditions. Preferably, the primer is bound to the primer-binding site by a sense-antisense interaction without base mismatches. In addition, the primer-binding site may also comprise one or more modified bases such as abasic sites which do not form hydrogen bonds.

### Primer-binding region

The term "primer-binding region" refers to a region within an adapter comprising a sequence or base composition representing a primer-binding site, or give rise to a primer binding site by copying and/or conversion. The primer-binding region of an adapter may be single-stranded or double-stranded and may comprise more than one primer-binding site. Preferably, the primer-binding region comprises one or more universal or directly convertible base(s).

### Nucleic acid triphosphate

The term "nucleic acid triphosphate" or "nucleotide" refers to any of the naturally occurring ribonucleotides (ATP, CTP, GTP, and UTP), deoxyribonucleotides (dATP, dCTP, dGTP, TTP, and dUTP), their derivatives, and combinations thereof. Derivatives may include but are not limited to base modifications of nucleic acid triphosphates such as 5-methyl-cytosine, 7-deaza-guanine, 5-bromo-uracil, 8-oxo-guanine, 2-aminopurine, N6-Methyl-adenine, Cy5-uracil, Cy3-uracil, Cy5-cytosine, and Cy3-cytosine. Furthermore, sugar modifications may include locked nucleic acids (L-NTP), unlocked nucleic acids, (U-NTP), 2'fluoro-NTP (2F-NTP), 2'O-methyl-NTP (2OMe-NTP), 2'azido-NTP, arabinose-NTP (ara-NTP), and dideoxides (ddNTP). Also phosphate modifications of nucleotides such as alpha-phosphorthioates may be included.

### Universal base

The term "universal base" refers to a base or analogue thereof which is able to pair with more than one of the four natural bases or does not lead to a mismatch which destabilises the helical structure of a nucleic acid duplex. A universal base may interact with its opposite base by hydrogen bonds, form hydrophobic stacking interaction with adjacent bases. In the case of abasic sites, no direct interaction with a base in the opposite strand is necessary. Base interactions can occur by Watson-Crick or Hoogsteen pairing. Preferred universal bases are nucleotides comprising inosine (hypoxanthine), xanthine, oxanine, 8-oxo-guanine, nebularine, 3-nitropyrrole, 5-nitroindole, 4-methylindole, O4-methyl-thymidine (O4mT), O4-ethyl-thymidine (O4eT), 6H,8H-3,4-dihydro-pyrimido[4,5-c][1,2]oxazin-7-one (P), N6-methoxy-2,6-diaminopurine (K), and 5-fluoroindole bases, pyrrolidine, and nucleotides with missing bases (abasic sites) such as dSpacer (1',2'-dideoxyribose), and other spacers such as diethyleneglycol (DEG), and butanol (C4 spacer). More preferred are universal bases such as inosine, 8-oxo-guanine and abasic site, that can be copied by a polymerase with a preference for only one of the naturally occurring bases.

### Convertible base

The term "convertible base" or "directly convertible base" refers to a base or analogue thereof which can be directly converted without a copying step to another base with a different coding property.

Convertible bases are preferably modified and placed in predefined sequences or oligonucleotides such as adapters. Such specifically convertible bases may comprise groups masking one or more of the positions in a base that are able to form hydrogen bonds with an opposite base in Watson-Crick and/or Hoogsteen basepairings. Such masking groups may be simple alkyl groups and/or protection groups known from chemical synthesis. Preferably, the protection group for amine is an hydrazine (N2), resulting in an azido-group (-N3), for oxygen and sulfur groups an O- or S-azidomethyl group, respectively. N3-protection groups can be cleaved off in a Staudinger-reaction by strongly reducing reagents such as TCEP. Alternatively, the convertible base may have groups that can be selectively modified with masking groups. Yet another directly convertible base is a cleavable base that gives rise to an abasic site by conversion. Alternatively, an abasic site may comprise a modification at the sugar moiety that allows specific attachment of a base such as 1-ethynyl-dSpacer that can be reacted with an azide-bearing base analogue by click chemistry. The base conversion can be preformed enzymatically, chemically and photochemically. Preferably, the direct conversion reaction is compatible with enzymatic activities such as ligation or nucleic acid polymerase reactions and the respective buffers. Preferred directly convertible bases are nucleotides comprising O4-methyl-thymidine, O6-methyl-guanine,S4-methyl-thio-uridine, S4-methyl-thio-thymidine, S6-methyl-thioguanine, N1-methyl-adenine, N3-methyl-adenine, C8-methyl-guanine, N2,3-etheno-guanine, O6-methyl-hypoxanthine, O4-ethyl-thymidine, O6-ethyl-guanine, S4-ethyl-thio-uridine, S4-ethyl-thiothymidine, S6-ethyl-thio-guanine, N1-ethyl-adenine, N3-ethyl-adenine, C8-ethyl-guanine, O6-ethyl-hypoxanthine, S4-thio-uridine, S4-thio-thymidine, S6-thio-guanine, 1-ethynyl-dSpacer (abasic site with an alkyne group), and 1-thiol-dSpacer (abasic site with a thiol group).

### Detailed description of certain embodiments

The invention relates to a method comprising single or at least partially double-stranded adapters comprising sequences with base modifications that are ligated to nucleic acid fragments and converted to generate asymmetric ends for specific recognition. The method based on two main sequence conversion mechanisms, a direct conversion of specific bases and an indirect conversion by copying, wherein both mechanisms may be combined.

### I) Indirect sequence conversion

The indirect sequence conversion mechanism for universal bases is based upon differential reading and/or annealing to bases that are incorporated in one or both of the ligated universal base adapter strands. Universal bases can bind to more than one base in the opposite strand without mismatch and can be converted to a different base by a polymerase or primer binding. For instance, abasic sites and other universal bases that do not form hydrogen bonds can be converted to A or C in the opposite strand depending on the polymerase. Furthermore, the bias of a polymerase can be forced toward incorporation of a desired base by changing the balance of nucleotides supplied in the reaction.

A convertible adapter with universal bases of which both strands are ligated to a nucleic acid fragment preferably comprises two annealed strands comprising a double-stranded region. The double-stranded region comprises at least a ligatable end. The first of the annealed strands comprises a primer binding site and a ligatable 5' end. The primer-binding site may comprise one or more universal bases and thus may give rise to a new primer binding site after conversion in an primer extension reaction. The second annealed strand of the universal adapter may comprise a precursor primer binding site in the primer-binding region and a ligatable 3' end. The second strand may comprise one or more universal bases in a precursor primer binding site which may also give rise to a new primer binding site after conversion by a polymerase. The primer-binding region of the convertible adapter comprises both the primer binding site in the first adapter strand and if present, the precursor primer binding site in the second strand. The two annealed strands may be joined at their ends by a linker or a nucleic acid loop. Such loops can be useful to generate a covalently closed double-stranded ligation product that can be amplified by a rolling circle mechanism or represent a template for single molecule sequencing according to the single molecule real time (SMRT) DNA sequencing technology. However, it is preferred to use two discontinuous strands that are annealed to form an at least partially double-stranded adapter according to the disclosure.

Universal bases are converted by two different copy mechanisms depending on their incorporation in one of the strands of a ligated adapter. The primer-binding strand is ligated to the nucleic acid fragment via its 5' and has a free, preferably blocked and non-ligatable, 3'-terminus. The primer-binding site of the first strand is not copied by a polymerase, but converted to any sequence depending on the first primer that anneals to the first strand at the primer binding site of the adapter molecule (see Fig. 1a). Thus, modifications that cannot be copied by polymerases but pair with opposite bases in the primer are compatible with primers and fall within the scope of the invention. Contrarily, the second strand of the adapter which is ligated via the 3' end and having a free 5'-terminus does not anneal to a first primer and universal bases need to be converted by a polymerase in order to provide a second but different specific primer binding site (see Fig. 1b). Thus, it is preferred that modifications that are incorporated in the second adapter strand in the precursor primer binding site can be copied and/or converted by a polymerase. Although It is possible to incorporate universal base(s) in only one of the two adapter strands, it is preferred to incorporate universal bases in both adapter strands in order to more efficiently generate at least two new and dissimilar primer binding sites. This strategy is even more efficient, if the primer comprises different bases opposite the universal bases in the primer-binding site than in the adapter and the polymerases converts the bases in the other adapter strand to yet another base (see Fig. 1c). The new primer binding sites may not overlap with respect to the original primer binding site in the adapter. In case of non-overlapping primer-binding sites, it is preferred to prevent polymerase extension beyond the precursor primer-binding site on the second strand of the adapter by a blocking modification, or only a single-stranded region for one of the primer-binding sites (see Fig. 2). The outer primer-binding site (site A in Fig.) may be used only on the first copying steps in the case that modified bases in the primer-binding site require a very low annealing temperature that can lead to unspecific hybridisation. However, it is generally preferred to reduce the size of the adapter by using overlapping primer binding sites.

The universal base conversion can also be conducted by differently biased polymerases after ligation. A different incorporation bias for one or more universal base(s) can be exploited to generate different primer-binding sites at both ends of a ligated nucleic acid fragment in subsequent amplification cycle(s). This can be achieved by using polymerases with different incorporation bias in the first and following amplification steps. Ideally, such polymerases follow different rules in non-templated base incorporation that will convert abasic sites and/or universal bases with hydrophobic stacking interaction accordingly. Preferably, the first DNA polymerase is thermolabile and the second polymerase is a hot-start polymerase. For example, the first polymerase is human DNA polymerase beta (C-rule) and the second is a hot-start Taq DNA polymerase (A-rule). Thus, the first extension step is performed by the first polymerase at a lower temperature, and after heating all further amplification steps are performed by the second polymerase. Preferably, the first two extension cycles are preformed longer than in following cycles in order to allow efficient translesion synthesis to occur.

### Convertible adapter design

Depending on the desired adapter sequences, different approaches can be chosen. In case that only one of the two asymmetric ends needs to yield a predefined sequence, the second strand is mainly converted to the desired sequence when copied and the primer binding strand of the adapter is sufficiently different from the copied second strand to allow specific second primer binding and extension. The primer binding strand of the adapter can be altered to incorporate modifications that tolerate base pairing or wobble which preferably a higher melting temperature than the same sequence of the upper strand and/or its copy.

In some cases, both ends of the ligated adapter need to result in predefined sequences to be compatible with existing formats such as those already used in next generation sequencers (see SEQ ID NO:1-7), or for cDNA transcription/expression using specific promoter and/or terminator sequences (see SEQ ID NO:8-11). One way to generate such defined sequences is to generate an adapter as described above for the first converted sequence and simply add the required secondary sequence as an extension. This extension can be made by fusing the sequence 3' to the lower adapter strand, or by using a primer that comprises the desired region to be added by polymerase extension. Yet another approach can be chosen to yield defined ends in which a common, unchanged sequence is present that is directly ligated to the template. In order to allow differential amplification of the end by two different primers, the first primer binding strand comprises several modifications and a unique single stranded overhang that preferentially allows binding to only one primer under stringent conditions.

The universal base can be chosen depending on which sequences need to be generated after conversion. For instance, an inosine can be used in the double stranded region of the adapter as a matched I·A base pair that can be converted to a mismatching G (via incorporation of a C in the opposite strand) and A which does not pair at all. Slightly different choices can be made for oxanine or xanthine by matching the universal bases with T which can be converted to a mismatching G (via incorporation of a C in the opposite strand) and T. 8-oxoguanine can match with A and can be converted to yield a mismatching G·A. Other universal bases that use hydrophobic stacking rather than hydrogen bonds for pairing can be used as wildcards to replace any base but T or U when using a polymerase that follows the "A-rule". Since some polymerases such as human DNA polymerase beta, follow the "C-rule", any base but G can be replaced in a similar manner to yield converted sequences that mismatch in the primer binding site of the second strand. Polymerase mutants can be also selected for following a different rule. However, it is preferred that polymerases lack a proofreading function such as 3' exonuclease and/or lyase activity. Thus, abasic sites can be used according to the rule of the polymerase to be used in extension and/or amplification. In contrast to other universal bases it is preferred to use only one abasic site in combination with neighbouring bases in order to keep a duplex structure under annealing and ligation conditions. Abasic sites should not be placed directly at the terminal base ligation site. Preferably, the abasic site is placed 2 bases and more preferably more than 3 bases away from the ligation site. Additional guidance for using universal bases for sequence conversion and compensating for lower annealing temperature is given in further detail below and in Table 1.

### Requirements for inosine

As a universal base, inosine can essentially pair with all bases. However, inosine pairs with the following bias: I·C > I·A > I·T, I·G > I·I. As all polymerases incorporate C opposite of inosine, the adapter sequence can be exchanged at essentially all G to I and still yield the same sequence after conversion. Both strands can be modified with inosine and yield very distinct primer binding sequences for an efficient PCR. Preferably, the base opposite to inosine in the universal base adapter is exchanged to an A to preserve a double-stranded conformation while also maintaining a high melting temperature.

### Requirements for abasic site

Abasic sites can form natural duplex structures without mismatches or bulges. Especially, pyrimidines opposite the abasic sites result in stable helical conformations. Most polymerases follow the so-called "A-rule" which yields incorporation of dATP opposite to abasic sites. Thus, it is preferred to incorporate a guanine opposite to an abasic site in the adapter in order to provide a sequence conversion, especially when copied by a polymerase. However, at temperatures typically used for ligation reactions, all natural bases are well tolerated opposite abasic sites. Still, berberine alkaloids, flavines or other abasic site-binding small molecules can enhance the duplex structure and elevate melting temperature. This also applies to purines in the opposite strand. As it is known that berberine and related molecules do not inhibit polymerases, they can be used in the amplification reaction and enhance sequence conversion of abasic sites without introducing deletions.

### Compensating for low melting temperature

Although it is preferred to use bases with higher melting temperatures in the opposite strand of universal bases, it is possible to compensate for lower affinities or melting temperatures. It is known that different nucleic acid backbones result in different melting temperatures even with the same base composition. For example, PNA > LNA > RNA > DNA is the general order melting temperatures of different nucleic acids annealed to a given DNA sequence. As PNA cannot be copied by a polymerase and LNA requires specific polymerase mutants, it is preferred to restrict such backbone modifications to the primer binding site of the primer binding strand of the adapter. In addition, some modified bases can be used to increase the melting temperature as well. Non-limiting examples are 2,6-diaminopurine (2-amino-dA), 5-methyl dC, and Super T (5-hydroxybutynyl-2'-deoxyuridine). These base modifications are faithfully copied by most polymerases and can therefore be incorporated instead of the natural bases in both of the adapter strands, preferably adjacent to a potentally destabilising universal base.

**Table 1: Non-limiting examples for universal bases and their properties**

| base | Mode of interaction | Pairing with opposite base | Opposite base incorporation | Polymerase example |
|---|---|---|---|---|
| inosine | Hydrogen bonds | C > A > T, G | C | Taq, Pfu |
| oxanine | Hydrogen bonds | C > T > A >G | C > T | Taq |
| xanthine | Hydrogen bonds | T > G > A, C | C > T, G | Taq |
| 8-oxo-guanine | Hydrogen bonds | A, C | C > A | Pfu |
| nebularine | Hydrogen bond | All four | T | Klenow fragment |
| 3-nitropyrrole | Hydrophobic stacking | All four | A | Taq (A-rule) |
| 5-nitroindole | Hydrophobic stacking | All four | A | Taq (A-rule) |
| 4-methylindole | Hydrophobic stacking | All four | A | Taq (A-rule) |
| P | Hydrogen bonds | G, A | G, A | Taq |
| K | Hydrogen bonds | C, T | T > C | Taq |
| abasic site | None | All four | A > deletion | Most polymerases (A-rule) |

### II) Direct sequence conversion

Universal bases can be introduced after the ligation step by site-specific modification of bases in the primer binding region. For example, uracil can pair with adenine (U·A) in a duplex which is recognised by Uracil-DNA glycosylase, also known as UNG or UDG, leading to excision of the uracil and generation of an abasic site. By using a polymerase not following the "A-rule", a different pair other than T·A will be generated by conversion. The deglycosylated uracil site (abasic site) can also be used in the first strand of the universal base adapter to bind to any base in a hybridised primer. Many other bases that specifically pair with opposite bases can be excised specifically to yield abasic sites. However, it must be considered that this may also convert modified bases present in the ligated nucleic acid fragment of a given sample. It is also possible to utilise a chemically or photocleavable base which is removed after ligation. For example, it is known that alkylated bases such as N7-alkylguanine are much more susceptible to deglycosylation than natural bases.

Alternatively, a base is directly converted to a different base by an enzyme, a chemical or photochemical reaction. A preferred approach is to differentially expose groups of a base capable of forming hydrogen bonds with other bases by either Watson Crick or Hoogsteen interactions in order to alter the coding properties. This can be achieved by using enzymes that specifically recognise and change groups of the base such as activation-induced cytidine deaminase (AID) and APOBEC1 that convert cytosine into uracil.

Yet another, more specific enzymatic conversion is possible using O4-methylthymidine (O4mT) or O4-ethylthymidine (O4eT) which can pair with G and is preferably read as a C by polymerases. It has been discovered that O4eT is more useful as it is more efficiently read as a C despite a lesser bypass efficiency by most polymerases. Another useful alkylated base according to the invention is O6-methylguanine (O6mG) which pairs well with the natural bases T or U and is read as a A by polymerases. The alkyl-groups can be removed enzymatically using Ogt or Ada methyltransferases in order to convert O4mT or O6mG into a normal bases (T and G, repsectively). Especially the Klenow fragment mutant M747K of Taq DNA polymerase (KlenTaq M747K) is preferred for efficient incorporation of a non-cognate base opposite of an alkylated base and may also be chosen to convert alkylated thiol-bases disclosed below. In addition, Vent exo- is also able to preferentially incorporate T opposite of O6mG even at 37°C. Also Bacillus stearothermophilus DNA polymerase I large fragment (Bst) effiently prefers incorporating C vs. T opposite of O6mG.

However, it is preferred to use a chemical or photochemical method to convert bases by differential exposure of functional groups. One example for chemical conversion of bases applied in so-called bisulfite sequencing. The nucleic acid in a sample is exposed to bisulfite to determine its pattern of methylation. Treatment of DNA with bisulfite converts cytosine residues to uracil, but leaves 5-methylcytosine residues unaffected. Thus, bisulfite treatment introduces specific changes in the DNA sequence that depend on the methylation status of individual cytosine residues, yielding single-nucleotide resolution information about the methylation status of a segment of DNA. Surprisingly, it was discovered that it is possible to design a perfectly matched adapter molecule that can be ligated to nucleic acid fragments and treated with bisulfite to yield new sequences useful for generating an asymmetrically tagged nucleic acid fragment. By using 5-methyl C (mC) in one strand of the adapter, the sequence remains unaltered, whereas the all C are converted to A in the other strand. Preferably, an adapter suitable for bisulfite sequencing comprises at least two non-methlyated cytosines. By introducing I in the non-5-methyl C strand, preferably opposite A, the lower melting temperature by C to A conversion can be compensated. Alternatively, other modifications for higher melting temperature can be employed as well. Such specifically designed directly convertible adapters provide the advantage that only completely converted nucleic acid fragments are amplified. Thus, incompletely bisulfite-treated nucleic acid fragments that may cause false positives in methylation assignment can be avoided.

Another more adapter-specific modification for post-ligation base conversion can be made by incorporating 6-thioguanine (6-tG) pairing with C (6-tG·C) in an adapter which can be methylated preferably after ligation to form S6-methylthioguanine (m6-tG). Specific 6-tG methylation can be achieved chemically using S-adenosylmethionine or other alkylating agents. The methyl-group of me6-TG acts as a protection group that hinders the formation of a hydrogen bond with an cytosine.

As polymerases incorporate T opposite of me6-tG, a specific base exchange can be introduced after conversion (m6-tG·T versus G·C) to generate an asymmetrically tagged nucleic acid fragment according to the invention. Alternatively, the alkylated form of 6-tG (preferably with cyanoethyl-group) is used for coding first and deprotected by NH4OH in the presence of NaSH. However, it is preferred to use a protection group forming a disulfide bond with the 6-thiol group. Preferably, the thiol group is reacted with N-((3-chloropropyl)thio)phthalimide to attach a -SCH2CH2CH2CI protection group. The protection group can be removed with reducing agents such as TCEP, DTT or mercaptoethanol which are compatible with many enzymes including polymerases.

The commercially available building block 4-thio-uridine (4-tU) can be treated in basically the same manner as described above for 6-tG. Thus, the uridine derivative can also be switched from a C-analogue to a T-analogue and back depending on the reaction conditions. However, 4-tU (and 6-tG) can also be cross-linked with a reactive group upon irradiation in the UVA range (315-400 nm). In the presence of oxygen it can react to form guanine-6-sulfinate and guanine-6-sulfonate or form crosslinks with adjacent or opposite bases. Although crosslinks can be partially avoided by reducing agents, it is preferred to use deprotection of groups forming disulfide bonds to change the coding properties of thiolated bases. Typical protection groups for bases in oligonucleotide synthesis are N-benzoyl (Bz), N-acetyl (Ac), N-isobutyryl (iBu), N-phenoxyacetyl (PAC) and N-tert-butylphenoxyacetyl (tBPAC), of which Ac and iBu are preferred as less interfering in functional base pairing. Many ways for base deprotection are known. Aqueous methylamine effectively cleaves all of these protecting groups from the exocyclic amines. Ethanolic ammonia shows the highest selectivity between standard protecting groups (Ac, Bz, iBu) and fast-deprotecting groups (PAC, tBPAC). In addition, some of these protection groups can be cleaved off enzymatically. For example, N-phenylacetyl-groups can be removed by acetyl esterase or penicillin G acylase.

Some examples for useful base conversions are listed in Table 2 based on naturally occurring examples. However, many protection groups are known which can mask one or more functional groups of a base. Especially oxygen groups may be simply protected by azidomethyl groups that can easily be cleaved off by TCEP. Notably, photochemical cleavage of sensitive protection groups is a favoured method to change the coding properties of a given base. The skilled person would be aware of methods for differentially exposing groups capable of forming hydrogen bonds in a base to alter the coding properties by enzymatic, chemical or photochemical treatment.

In order to increase the efficiency of asymmetric end generation by post-ligation base conversion, the adapter may comprise one or more universal base(s) in the primer binding region before the ligation.

**Table 2: Non-limiting examples for convertible bases**

| base | Conversion method | Opposite base incorporation | Incorporation after conversion | Polymerase example |
|---|---|---|---|---|
| cytosine | Deamination, enzymatic, bisulfite | G | T | Most polymerases |
| Lesions eg. 8-oxo-guanine | Deglycosylation, enzymatic | T, C | A (A-rule) | Most polymerases |
| O4-methyl-thymidine | Chemical, enzymatic (Ada, Ogt) | G > A | A | In vivo (human cells) |
| O6-methylguanine | Chemical, enzymatic (Ada, Ogt) | A > C | C | KlenTaq M747K, Bst exo-, Vent exo-, Klenow fragment exo- |
| S4-methyl-thiouridine | Chemical | G > A | T | In vivo (human cells) |
| S6-methyl-thioguanine | Chemical | A > C | C | Klenow fragment exo- |
| N1-methyladenine | Enzymatic (AlkB) | A | T | In vivo (human cells) |
| N3-methyladenine | Enzymatic (AlkA) | A | T | In vivo (human cells) |
| C8-methylguanine | Enzymatic (AlkA) | G | C | In vivo (human cells) |
| N2,3-ethenoguanine | Enzymatic (AlkA) | T > C | C | In vivo (human cells) |
| O6-methylhypoxanthine | Chemical, enzymatic (Ada, Ogt) | T | C | Klenow Fragment, Taq, Tth |

### Ligation methods

The universal base adapter may be blunt ended at the ligatable end. In this case, the nucleic acid fragments to be ligated have to be generated with blunt termini as well. This can be achieved by 3' polymerase fill in reactions, 3' single-strand digests or by restriction enzymes that generate blunt ends. The person skilled in the art will be familiar with multiple protocols to generate nucleic acid fragments with blunt ends from nucleic acid samples for subsequent ligation. However, it is preferred to generate nucleic acid fragments with sticky ends that are much more efficient in ligation with complementary overhangs at adapter ends. Single base overhangs can be introduced to blunt ends by using the non-templated base-adding activity of polymerases such as Taq DNA polymerase or Klenow fragment exo-. Polymerases following the A-rule most efficiently add single-base A-overhangs. Such A-tailed nucleic acid fragments can be ligated to adapters with single-base T or U overhangs. However, restriction enzymes can also be used to generate defined or even random overhangs that can be ligated to complementary overhangs at adapters.

Universal base adapter ligation is preferably performed using a ligase such as the T4 DNA ligase. Alternatively, the adapter can be reacted with a site-specific topoisomerase, preferably by a Vaccinia virus topoisomerase. In order to generate an universal base adapter with a 3' covalently linked topoisomerase, it is preferred to introduce an enzyme-specific target site outside the primer-binding region in the adapter. By cleavage at the target site a single-stranded overhang is released and the enzyme becomes covalently linked at the ligatable end at the 3' end. The methods how to generate such topoisomerase adapters for ligation are well known to the skilled person, especially those based on Vaccinia virus topoisomerase. The nucleic acid fragment to be ligated may be blunt-ended or comprise short 3' overhangs. Importantly, the 5' end of the nucleic acid fragment has to remain unphosphorylated or otherwise unblocked for ligation to the 3'-end of the ligatable end of the universal base adapter. As the topoisomerase ligates only one of the two strands of the universal base adapters, the other strand may need to be ligated by another method. In order to fill potential gaps between the 5' end of the first strand of the adapter and the 3' end of the nucleic acid fragment, a polymerase without strand displacing activity and preferably without 3' exonuclease activity may be used. A non-limiting example for a suitable DNA polymerase to filling such gaps is T4 DNA Polymerase. The remaining nick can be sealed by a ligase provided that the 3' end of the nucleic acid fragment is unphosphorylated and the 5' end of the adapter is phosphorylated.

Alternatively, the base conversion is performed with an adapter requiring only one adapter strand to be ligated as disclosed above for post-ligation base conversion methods. This allows skipping the enzymatic filling and nick sealing reactions.

Transposases have been applied previously for adapter ligation by strand invasion. Especially useful transposases are the Tn5 transposase and the bacteriophage Mu transposase which are commercially available for ligating tags to nucleic acid fragments for amplification and/or sequencing. Transposases recognise specific double-stranded target sites such as 5'-AGATGTGTATAAGAGACAG-3' for Tn5 transposase that are bound to the enzyme. The adapter for transposase ligation comprises the double stranded target site and a 5' extension thereto comprising a primer-binding region. In the case of Tn5 adapter, it is preferred that the 5' extension comprises a sequence which can be converted to yield 5'-GCCTCCCTCGCGCCATC-3' and optionally 5'-GCCTTGCCAGCCCGCTC-3' by using universal base incorporation and/or post-ligation base conversion. Alternatively, the transposase target-sequence itself can be modified to include universal bases or convertible bases in order to generate asymmetric ends. Known variable bases in the target-sequence can be used to generate a new adapter wherein the ligation site may completely overlap with the primer-binding site.

Another method for ligating adapters to given nucleic acid fragments is to use enzymatic recombination. However, target-sequences for recombinases are non-random and may severely compromise a statistical adapter ligation approach. Instead, it is preferred that a recombination site in an adapter is used for circulation of ligated nucleic acid fragments for rolling circle amplification or mate-pair sequencing.

As target sites for topoisomerases, transposases and recombinases become directly attached to the nucleic acid fragment by enzymatic ligation, they can also be considered as ligation sites or ligatable ends.

### Additional functional sequences in an adapter

Other than the above mentioned primer-binding sites, topoisomerase target sites, transposase target sites and recombination sites, it is preferred that tags or barcodes are introduced as well. Tags or barcodes represent short sequences (less than 50 bases, preferably less than 20 bases) that can be used to assign a given sequence to a specifically tagged sample. This allows for economic multiplexing of samples in sequencing reactions. In some cases, random sequences may be used instead of defined sequence tags as well.

So-called key sequences may also confer the advantage to evaluate the correctness of library generation and estimation of the correct start of the ligated nucleic acid fragment in sequencing.

For transcription and expression of nucleic acid fragments it is required to have promoter sites for specific RNA polymerase binding and initiation. Preferred RNA polymerases include bacteriophage T7 RNA polymerase, bacteriophage SP6 RNA polymerase and cyanophage Syn5 RNA polymerase and their respective promoter sequences (SEQ ID NO:9-11).

### Further important amplification parameters

Since some universal bases and directly convertible bases may lower the annealing temperature in a primer binding site and cannot always be rescued by compensating base modifications in adjacent bases, the annealing of the first primer at the first primer-binding site may be lower than in later amplification cycles. In order to optimise the amplification it is recommended to identify the specific annealing temperature empirically. After conversion, conventional primer annealing temperature calculations may be sufficiently correct. Thus, the sequences of the converted primer-binding sites will have a lower melting temperature when hybridised to each other than with the cognate primer. Preferably, the melting temperature of the converted primer binding sites is at least 2°C lower, more preferably 5°C lower, and most preferably lower than 10°C for the non-cognate versus cognate primer in order to achieve a highly specific priming in amplification and sequencing reactions.

In case only one or more bases are exchanged in the primer binding site, these are preferably exchanged at or close to the 3' end of the respective primer binding site. Several priming methods are known in the art that can result in specific priming despite little melting temperature differences. Generally, mismatch types within the three bases closest to the 3'end affect specificities of primers. In the third base, C·A and T·G belong to weak destabilization strength mismatches. The mismatches G·A, T·C, T·T, and C·C located at the fourth base away from the converted site belong to the strong destabilization strength mismatches. According to the combination rules, polymorphic efficiency between T·T (mismatch in 3'end of primer, strong destabilization strength) and C·A (weak destabilization strength) are typically higher than A·A (mismatch in 3'end of primer, medium destabilization strength) and C·A. However, oligonucleotide synthesis may not always be absolutely correct and can produce artefacts that may lead to amplicons without any nucleic acid fragment inserts. Furthermore, in order to make amplification more efficient by preventing end loop formation, it is preferred to add different 5' extensions to the primers that are not present in the adapter. In addition, mispriming can be avoided by providing a blocking moiety such as a dideoxy-terminated base at the 3' end of the primer that prevents extension. Only upon correct annealing, the blocking moiety is removed and the primer can be extended on the cognate template. Non-limiting examples for such template-specific unblocking is the introduction of cleavable moieties at the base(s) that differ(s) in the primer binding sites. Especially nucleic acid repair enzymes are suitable. For instance, a thermostable RNAseH (more specifically RNAseH2) and a single RNA base in the 3' region of the blocked primer can only cleave and unblock the primer if the primer is fully annealed. In addition, endonuclease IV can cleave 5' from an internal C3 spacer and that 3'-exonuclease activity of a thermostable polymerase can cleave off a 3'-C3 blocker when 3'ends of primers form a perfect Watson-Crick pairing with the templates. Similar approaches have been established for other modifications as well. However, it the proper enzyme should be chosen in order not to cleave any of the modified base(s) in the primer-binding region of the adapter.

### Workflow for adapter ligation and conversion

An example workflow for adapters with a single-stranded primer-binding region comprising directly convertible bases comprises several steps starting with a ligation step (see Fig. 3). The ligation can be performed without a second strand in the adapter. Optionally, a second strand can be annealed to the ligatable end to improve the ligation efficiency. The optional second adapter strand at the ligatable end can be simply removed, for example by raising the temperature for performing a first extension step. Alternatively, a strand-displacing polymerase is used that initiates from the ligated nucleic acid fragment 3' ends in the first extension step, or a base with a 5' exonuclease activity that can remove the strand by a nick-tanslation mechanism. Thus, the convertible bases in the primer-binding region are copied in a first copying step on the basis of their first coding property, giving rise to a first primer-binding site comprising a unique sequence. In the following step, the convertible bases are converted to their second coding property. By using a primer specifically binding to the first primer-binding site a second copying step can be performed to generate asymmetrically tagged nucleic acid fragments.

An example workflow for adapters with a single-stranded primer-binding region comprising universal bases can be performed by using at least two polymerases with different conversion bias (see Fig. 4). After ligation, the optional unligated strand is removed by heating or using a strand-displacing polymerase or a polymerase with 5' exonuclease activity. The first polymerase with a bias for a first base initiates from the ligated nucleic acid fragment 3' ends in the first extension step. Thus, the convertible bases in the primer-binding region are copied in a first copying step on the basis of the first polymerase bias, giving rise to a first primer-binding site comprising a unique sequence. By using a primer specifically binding to the first primer-binding site a second copying step is performed by a second polymerase with bias for a second base to generate asymmetrically tagged nucleic acid fragments.

By ligating two strands of the adapter to both ends of a double-stranded nucleic acid fragment, less steps are necessary for conversion. As disclosed for single-stranded adapter ligation above, different mechanisms for conversion are possible.

For example, a direct conversion method for adapters with a double-stranded primer-binding region comprising directly convertible bases may comprise several steps starting with a ligation step for attaching both strands to the double-stranded nucleic acid fragment (see Fig. 5). The sequence comprising the convertible bases in the primer-binding region is exchanged after ligation, thus generating the new primer-binding site(s). After only one primer annealing and extension step, asymmetrically tagged nucleic acid fragments are generated.

An example for an indirect conversion method for adapters with a double-stranded primer-binding region comprising indirectly convertible bases may also comprise several steps starting with a ligation step for attaching both strands to the double-stranded nucleic acid fragment. Depending on the placement of universal base(s), the conversion is not performed by polymerase alone, but also includes conversion by primer hybridisation and elongation (see Figs. 1a-c and 6). Generally, the same amount of steps are necessary for such a conversion. Two consecutive primer hybridisation and extension steps give rise to asymmetrically tagged nucleic acid fragments.

In addition to these examples, many combinations are possible such as using both convertible and universal bases and even polymerases with different bias for base incorporation. However, based on the disclosed properties of universal bases and convertible bases and guidance for their incorporation in adapter strands, the person skilled in the art will know how to design convertible adapters to arrive at a protocol for generating asymmetrically tagged nucleic acid fragments.

### Applications

The main application for universal base adapters according to the disclosure is ligation to essentially unknown nucleic acid fragments. Such fragments may be derived from living or dead organisms, or of synthetic origin. Preferred are nucleic acid samples of human origin to be used in diagnostics, theranostics and forensics. Libraries generated by universal base adapter ligations can serve as templates for nucleic acid amplification, transcription and translation. A library can be sub-cloned into a vector or introduced into a genome for in vivo studies or more preferably used for sequencing.

### Examples

### Convertible adapter design

A standard adapter for sequencing was chosen as a template to design a convertible adapter according to the disclosure. The conventional lonTorrent adapter comprises two different double-stranded subunits to generate asymmetrically tailed nucleic acid libraries:
Adapter P1
   5'- CCACTACGCCTCCGCTTTCCTCTCTATGGGCAGTCGGTGAT 3' 3' T*T*GGTGATGCGGAGGCGAAAGGAGAGATACCCGTCAGCCACTA-PO4 5'
   * indicates phosphorothioate modifications
Barcode Adapter A
   5'- CCATCTCATCCCTGCGTGTCTCCGACTCAGNNNNNNNNGAT 3' 3' T*T*GGTAGAGTAGGGACGCACAGAGGCTGAGTCNNNNNNNNCTA-PO4 5'
   For better comparison with overhang-based ligation, T-tailed Adapters were designed:
Adapter P1-T
   5'- CCACTACGCCTCCGCTTTCCTCTCTATGGGCAGTCGGTGATT 3' 3' T*T*GGTGATGCGGAGGCGAAAGGAGAGATACCCGTCAGCCACTA-PO4 5'
   * indicates phosphorothioate modifications and added T is undelined
Barcode Adapter A-T
   5'- CCATCTCATCCCTGCGTGTCTCCGACTCAGNNNNNNNNGATT 3' 3' T*T*GGTAGAGTAGGGACGCACAGAGGCTGAGTCNNNNNNNNCTA-PO4 5'

Two Primers are used in PCR for amplification of ligated adapters:
Full-length Primer P1
   5'-CCACTACGCCTCCGCTTTCCTCTCTATGGGCAGTCGGTGAT-3'
Full-length Primer A
   5'-CCATCTCATCCCTGCGTGTCTCCGACTCAG-3'
Shortened primer minA
   5'-CTGCGTGTCTCCGACTCAG-3'
Shortened primer minP1
   5'-CTCTCTATGGGCAGTCGGTGAT-3'

Incorporation of inosine (I) instead of G does not lead to a sequence change of a template in PCR. I can pair with A and therefore it is preferred to exchange G in the Adapter A sequence to I while exchanging the opposite C to A. A ligation with T-A overhangs is preferred over blunt ends due to higher efficiency. The following sequence was designed to meet the requirements for a convertible adapter:
Barcode Adapter IA+T: 5'- CCATCTCATCCCTICITITCTCCIACTCAINNNNNNNNGATT-3' 3'-T*A*GGIAGAGIAGGGAAGAAAAGAGGAIGAGIANNNNNNNNCTA-PO4 5'
Exchanged bases with respect to Barcode Adapter A are underlined.
Barcode Adapter oligo IA+T: 5'-CCATCTCATCCCTICITITCTCCIACTCAINNNNNNNNGATT 3'
Barcode Adapter oligonucleotide IA': 5' PO4-ATCNNNNNNNNAIGAGIAGGAGAAAAGAAGGGAIGAGAIGG*A*T

Metling temperatures were calculated based on standard values using the online tool 'OligoAnalyzer 3.1' 2016 (http://eu.idtdna.com/calc/analyzer). Surprisingly, the melting temperature of the sequence comprising inosine does not rate much lower than the unmodified sequence comprising G:
SEQUENCE
   5'- AIG AGI AGG AGA AAA GAA GGG AIG AGA IGG AT -3'
COMPLEMENT
   5'- ATC CCT CTC CTC CCT TCT TTT CTC CTC CTC CT -3'
LENGTH
   32
   GC CONTENT
   40.6 %
MELT TEMP
   64.9 °C

The calculated melting temperature of the converted strands is:
SEQUENCE
   5'- CCA TCT CAT CCC TGC GTG TCT CCG ACT CAG -3'
COMPLEMENT
   5'- CTG AGT CGG AGA CAC GCA GGG ATG AGA TGG -3'
LENGTH
   30
GC CONTENT
   60%
MELT TEMP
   65.9 °C

Primer A' can bind to the newly converted primer-binding site:
SEQUENCE
   5'- ATC CCT CTC CTC CCT TCT TTT CTC CTA CTC CT -3'
COMPLEMENT
   5'- AGG AGT AGG AGA AAA GAA GGG AGG AGA GGG AT -3'
LENGTH
   32
GC CONTENT
   50%
MELT TEMP
   63.6 °C

Primer A and Primer A' cannot form dimers and can bind specifically to their respective primer binding sites.
Full-length Primer P1
SEQUENCE
   5'- CCA CTA CGC CTC CGC TTT CCT CTC TAT GGG CAG TCG GTG AT -3'
COMPLEMENT
   5'- ATC ACC GAC TGC CCA TAG AGA GGA AAG CGG AGG CGT AGT GG -3'
LENGTH
   41
GC CONTENT
   58.5 %
MELT TEMP
   70.5 °C

The ISP beads may comprise the entire P1 sequence. However, the adapter does not require the entire sequence as it can be added during amplification using a tailed primer. Thus, a truncated sequence Delta5-P1 is proposed:
Delta5-P1 :
SEQUENCE
   5'- CCT CTC TAT GGG CAG TCG GTG AT -3'
COMPLEMENT
   5'- ATC ACC GAC TGC CCA TAG AGA GG -3'
LENGTH
   23
GC CONTENT
   56.5 %
MELT TEMP
   60.2 °C

Thus, a chimeric primer can be used to amplify the convertible I-adapter for merging the sequence with the P1 sequence for ISP beads:
Delta5P1A'
SEQUENCE
   5'- CCT CTC TAT GGG CAG TCG GTG ATC CCT CTC CTC CCT TCT TTT CTC CTA CTC CT -3'
COMPLEMENT
   5'- AGG AGT AGG AGA AAA GAA GGG AGG AGA GGG ATC ACC GAC TGC CCA TAG AGA GG - 3'
LENGTH
   53
GC CONTENT
   54.7 %
MELT TEMP
   70.4 °C

### Convertible adapter ligation and PCR

Experiments were conducted using a PCR fragment as a ligation template. The above described adapters were ligated to the fragment, ligation efficiency was evaluated by capillary electrophoresis and PCR and compared with the commercially available adapters in the IonXpress kit from Thermo Fisher.

### Material and Methods

### (1) A-tailing of template DNA framents

A 5'-phosphorylated 200 bp PCR fragment was used as a template. The A overhangs were generated by using 1 µg template DNA together with 2.5 u DreamTaq DNA Polymerase (Thermo Fisher), 2.5 µl DreamTaq DNA polymerase buffer, 0.02 mM dATP (final concentration) and adjusted to 25 µl volume with PCR grade water. The sample was incubated at 72°C for 20 min. The product was purified using the Agencourt AMPure XP System according to manufacturers protocol.

### (2) Convertible adapter hybridisation

Hybridisation of the convertible IA adapter was performed by adding matching molecular amounts of Barcode Adapter oligonucleotide IA+T and Barcode Adapter oligonucleotide IA'. A the sample was adjusted to a concentration of 20 pmol/µl and incubated at room temperature for 10 min. The same was performed for Adapter A-T and Adapter P1-T at a concentration of 25 pmol.

### (3) Ligation of adapters to template DNA

A ligation sample was set up with 2 u T4-DNA ligase (Thermo Fisher), 2 µl 10x T4 ligase buffer and 2 µl PEG. 20 pmol convertible IA adapter (25 pmol for each A and P1 adapters were added in the control) and 100 ng A-tailed template DNA. The sample volume was adjusted to 20 µl using PCRgrade water and incubated for 1 h at 20°C and heated for 10 min at 65°C.

### (4) Purification of ligation product

The Agencourt AMPure XP system was applied according to manufacturers protocol and 2 µl of the ligation product was analysed by capillary electrophoresis (Fragment Analyzer, AATI) using the High Sensitivity NGS Fragment Analysis kit according to manufacturers protocol.

### (5) PCR of ligation products and analysis

1 µl ligation product was used as a template in a PCR sample with 1 u DreamTaq DNA polymerase, 2.5 µl 10x DreamTaq DNA polymerase buffer, 0.2 mM dNTP (final concentration), 0.4 µM of respective primers and volume was adjusted to 25 µl by adding PCRgrade water. The Primer A and Delta5P1A' pair were used for the convertible IA adapter, and primer minA and primer minP1 pair for IonTorrent adapters. The PCR for convertible adapter was conducted using the following protocol: 95°C 3min; 4x [95°C 30sec, 56°C 30sec, 72°C 30sec]; 10x [95°C 30sec, 58°C 30sec, 72°C 30sec]; 72°C 5min. The PCR protocol for the IonTorrent adapter was 95°C 3min; 14x [95°C 30sec, 58°C 30sec, 72°C 30sec]; 72°C 5min. PCR analysis was performed by capillary electrophoresis using the dsDNA 905 Reagent kit according to manufacturers protocol.

### Results

According to capillary electrophoresis, the ligation efficiency of the convertible adapter was 3-fold higher than the IonTorrent adapters with T-overhang (see Fig. 7a). The PCR efficiency was estimated to be 10-fold higher (see Fig. 7b). This result is consistent with an efficient PCR of convertible adapter-ligated fragment versus the IonTorrent adapters of which only 50 % can be amplified.

### Efficiency of convertible adapters

In order to determine the efficiency of the convertible adapter versus the standard adapter, ligation experiments were made. The protocol followed the steps (1) to (4) of the previous chapter for the convertible adapter and steps (2) to (4) for the standard adapter with varying PCR fragment concentrations. The absolute concentrations were determined by droplet PCR using a QX200™ Droplet Digital™ PCR System (Bio-Rad). The resulting values for the standard (blunt end) adapter, convertible adapter with both blunt and T-overhangs were compiled in Fig. 8. The convertible adapter outperformed the standard adapter by more than an order of magnitude, especially at low input concentration.

### Preparation of sequencing library

Genomic DNA from an E. coli strain was used to prepare a shotgun sequencing library. The workflow comprises typical steps such as genomic DNA fragmentation using ultrasound, end repair and adapter ligation. The convertible adapter requires ligation of both strands which was facilitated by T/A overhangs to prevent adapter dimer formation. Standard adapter was used without any barcode as provided by the IonXpressPlus gDNA Fragment Library Preparation Kit. The convertible adapter was applied comprising a barcode for distinction in a sequencing experiment. The individual steps for general DNA preparation and the individual ligation with the different adapter formats are outlined in the materials and methods section below.

### Materials and Methods

Oligonucleotides for preparation of convertible adapter IA+T with barcode (*designates phosphorothioate linkages):
Ada142dT_BC2 5'-CCATCTCATCCCTICITITCTCCIACTCAITAAGGAGAACGATT
Ada142pos_BC2 5-PO4-ATCGTTCTCCTTAAIGAGIAGGAGAAAAGAAGGGAlGAGAIGG*A*T

Genomic DNA preparation steps:
(1) Fragmentation
   The input for fragmentation was 3µg genomic E. coli DH10B DNA. A use Bioruptor was chosen for fragmentation (3x 15min ultrasonic treatment)
(2) Purification
   Purification was performed with PureLink PCR Purification Kit (Thermo Fisher Scientific)
(3) End repair
   End repair was performed with 1 µg fragmented DNA with End-Repair-Enzyme and End-Repair-Buffer, reagents are part of IonXpressPlus gDNA Fragment Library Preparation Kit (Thermo Fisher Scientific)
(4) Purification
   1.8x volumes AMPureXP beads (Beckman Coulter) were applied for DNA purification and the yield of end-repaired DNA was 970 ng. Convertible adapter library generation steps:
(5) A-Tailing
   A n amount of -460 ng end-repaired fragmented DNA of step (4) was applied for A-tailing with Klenow-Fragment exo- (NEB).
(6) Purification
   1.8x volumes AMPureXP beads (Beckman Coulter) were used for purification and the yield of A-tailed DNA was determined as 312.5 ng.
(7) Convertible adapter ligation
   50ng A-tailed DNA was mixed with 75 pmole convertible adapter, 0.4 Unit T4 DNA Ligase, 2 µl reaction buffer, 2 µl PEG and 13.1 µl H2O. The mix was incubated for 20min @25°C.
(8) Size selection
   AMPureXP beads (Beckman Coulter) were used for a two sided size selection by which small and large fragments were sequentially depleted. The first bead selection was performed with 0.7x volumes, and the second bead selection 0.15x volumes. Elution was performed with 30 µl LowTE buffer.
(9) Ligation product amplification
   2 µl ligation product of step (8) were mixed with 2.5 µl 10x DreamTaq Buffer, 0.5 µl dNTPS (10 mM), 1.5 µl SeqPrimerSet (10 µM), 1Unit DreamTaq DNA Polymerase and 18.3 µl H2O (Thermo Fisher Scientific). Amplification was performed by PCR as follows: 1min @ 98°C, [15sec @ 98°C, 15sec @ 61°C, 15sec @72°C]x15, 5min @ 72°C, hold 4°C
(10) Purification
   For final purification, 1.0x volumes AMPureXP beads (Beckman Coulter) were applied. A total yield of 76 ng library was observed.

Standard adapter library generation steps:
(11) Library generation
   A total of 50 ng end-repaired fragmented DNA from step (4) was applied for Library preparation using the IonXpressPlus gDNA Fragment Library Preparation Kit (Thermo Fisher Scientific). The 50 ng DNA was mixed with 10 µl 10x Ligase Buffer, 2 µl Adapters, 2 µl dNTP Mix, 51 µl Nuclease-free Water, 2 µl DNA Ligase, 8 µl Nick Repair Polymerase and incubated for 15 min @ 25°C and 5 min @ 72°C.
(12) Size selection
   AMPureXP beads (Beckman Coulter)were used for two sided size selection. The first bead selection was performed with 0.7x volumes and the second bead selection with 0.15x volumes. Elution was achieved with 30 µl LowTE.
(13) Ligation product amplification
   2 µl ligation product from step (12) were supplied with 100 µl Platinum PCR SuperMix High Fidelity, 5 µl Library Amplification Primer Mix and 23 µl H2O; reagents are derived from IonXpressPlus gDNA Fragment Library Preparation Kit (Thermo Fisher Scientific). The PCR amplification protocol was: 5min @ 95°C, [15sec @ 95°C, 15sec @ 58°C, 1min @ 70°C]x15, hold 4°C.
(14) Purification
   1.0x volumes of AMPureXP beads (Beckman Coulter) were applied for purification and the total yield was determined as 9 ng.

Final preparation steps before sequencing:
(15) Library evaluation
   The library size and concentration was determined by capillary gel electrophoresis. The concentration of the standard library was 1869,6 pmole/l and the convertible adapter library was 18186,791 pmole/l.
(16) Final sequencing library generation step
   Both libraries were mixed equimolar and diluted to a total of 100 pM for sequencing by an IonTorrent PGM sequencer.

### Results and Discussion

The size distribution of both library preparations after PCR is shown in Fig 9. The size of the libraries is mainly down to the less exact method of size selection by Ampure beads. The yield of the library preparation using the convertible adapter is consistently about 10-fold higher than with the standard adapter. The library mix from step (16) was sequenced according to manufacturer's protocol for Ion PGM™ Hi-Q™ View OT2 Kit and Ion PGM™ Hi-Q™ View Sequencing Kit with an Ion 318™ Chip Kit v2 BC (size: 200 bp).

The standard sequencer software sorted the reads according to the adapter used on the basis of the barcode. The reads were mapped against the reference genome E. coli DH10B. It is obvious that the use of the convertible adapter IA+T (Fig. 10a) does not result in any bias with respect to read distribution or orientation when compared to the standard adapter (Fig. 10b) provided by the manufacturer's kit.

The obtained read lengths were evaluated as well as shown in Fig. 11a and b. The discrepancy between the read length distribution of the standard adapter versus convertible adapter IA+T can be attributed to the two sided size selection by beads. Fig. 9 demonstrates that the main peak of the of the convertible adapter library is at 313 bp with respect to the standard adapter library at 374 bp, which also shows that the size selection was not identical, but within the typical variation of the method based on Ampure beads. Of the entire fragment length, 43 bases can be attributed to the converted adapter sequence upstream of the P1 sequence. Furthermore, the barcode is only present in the convertible adapter, which also takes another 10 bases from the sequence reads that can be aligned with the genome. Thus, approximately 53 bases are truncated from the convertible library reads due to the strict size selection.

Taken together, the sequencing of the convertible IA+T adapter demonstrates the very high efficiency of library generation and otherwise identical performance with respect to the standard adapter in sequencing experiments.

### Library generation with universal bases in only one adapter strand

The power of conversion is sufficient for sequencing library generation even if only one strand is modified by universal bases. As an example, inosines were incorporated in the template (PF_Ad142v2BC4_r) strand, whereas the primer binding (PF_Ad142v2BC4_f) strand was kept identical to the adapter A sequence. This new convertible adapter IA/2 comprising a barcode was designed to be ligated to blunt ended fragments.
PF_Ad142v2BC4_f
5'-GCCAATCTAATCCATGAGTGTATCAGAATAAGTACCAAGATCGAT-3'
PF_Ad142v2BC4_r
5'-PO4-ATCGATCTTGGTACTIAITCIGAIACACICAIGGATIAGATIG-PO4-3'

The PCR of the new IA/2 adapter was performed with the standard A primer in combination with the primer binding to the converted sequence (PF_Apl_prim).
PF_Apl_prim
5'-GCCAATCTAATCCATGAGTGTATCAGAATAAG-3'

The convertible adapters IA and IA/2 were compared by ligation to a blunt ended PCR fragment according to the protocol of chapter "Convertible adapter ligation and PCR" steps (2) to (5).

The efficiency of the adapter with one inosine modified strand is comparable to the adapter with inosines in both strand (Fig. 12). Thus, an even more simple design is possible for a given sequence of interest by keeping this sequence constant in one of the strands while modifying the complementary adapter strand to yield a novel sequence by conversion.

### Sequence Listing

<210> 1
<211> 33
<212> DNA
<213> Artificial Sequence
<220> primer binding site
<223> sequencing primer binding site
<400> 1
   ACACTCTTTCCCTACACGACGCTCTTCCGATCT
<210> 2
<211>37
<212> DNA
<213> Artificial Sequence
<220> primer binding site
<223> sequencing primer binding site
<400> 2
   CGGTCTCGGCATTCCTGCTGAACCGCTCTTCCGATCT
<210> 3
<211> 33
<212> DNA
<213> Artificial Sequence
<220> primer binding site
<223> sequencing primer binding site
<400> 33
   GATCGGAAGAGCACACGTCTGAACTCCAGTCAC
<210> 4
<211> 34
<212> DNA
<213> Artificial Sequence
<220> primer binding site
<223> sequencing primer binding site
<400> 4
   GTGACTGGAGTTCAGACGTGTGCTCTTCCGATCT
<210> 5
<211> 32
<212> DNA
<213> Artificial Sequence
<220> primer binding site
<223> sequencing primer binding site
<400> 5
   CGACAGGTTCAGAGTTCTACAGTCCGACGATC
<210> 6
<211> 30
<212> DNA
<213> Artificial Sequence
<220> primer binding site
<223> sequencing primer binding site
<400> 6
   CCATCTCATCCCTGCGTGTCTCCGACTCAG
<210> 7
<211> 38
<212> DNA
<213> Artificial Sequence
<220> primer binding site
<223> sequencing primer binding site
<400> 7
   CTACGCCTCCGCTTTCCTCTCTATGGGCAGTCGGTGAT
<210> 8
<211> 23
<212> DNA
<213> Transposon Tn5
<400> 8
   TCAGAGATGTGTATAAGAGACAG
<210> 9
<211> 18
<212> DNA
<213> Bacteriophage T7
<220> promoter sequence
<400> 9
   TAATACGACTCACTATAG
<210> 10
<211> 23
<212> DNA
<213> Bacteriophage SP6
<220> promoter sequence
<400> 10
   ATTTAGGTGACACTATAGAAGNG
<210> 11
<211> 15
<212> DNA
<213> Cyanophage Syn5
<220> promoter sequence
<400> 11
   ATTGGGCACCCGTAA

### SEQUENCE LISTING

<110> Technische Hochschule Wildau
<120> Convertible Adapters
<130> bra107wo
<150> EP16188292.3
   <151> 2016-09-12
<160> 46
<170> PatentIn version 3.5
<210> 1
   <211> 33
   <212> DNA
   <213> Artificial Sequence
<220>
   <223> sequencing primer binding site
<400> 1
   acactctttc cctacacgac gctcttccga tct 33
<210> 2
   <211> 37
   <212> DNA
   <213> Artificial Sequence
<220>
   <223> sequencing primer binding site
<400> 2
   cggtctcggc attcctgctg aaccgctctt ccgatct 37
<210> 3
   <211> 33
   <212> DNA
   <213> Artificial Sequence
<220>
   <223> sequencing primer binding site
<400> 3
   gatcggaaga gcacacgtct gaactccagt cac 33
<210> 4
   <211> 34
   <212> DNA
   <213> Artificial Sequence
<220>
   <223> sequencing primer binding site
<400> 4
   gtgactggag ttcagacgtg tgctcttccg atct 34
<210> 5
   <211> 32
   <212> DNA
   <213> Artificial Sequence
<220>
   <223> sequencing primer binding site
<400> 5
   cgacaggttc agagttctac agtccgacga tc 32
<210> 6
   <211> 30
   <212> DNA
   <213> Artificial Sequence
<220>
   <223> sequencing primer binding site
<400> 6
   ccatctcatc cctgcgtgtc tccgactcag 30
<210> 7
   <211> 38
   <212> DNA
   <213> Artificial Sequence
<220>
   <223> sequencing primer binding site
<400> 7
   ctacgcctcc gctttcctct ctatgggcag tcggtgat 38
<210> 8
   <211> 23
   <212> DNA
   <213> Escherichia coli
<400> 8
   tcagagatgt gtataagaga cag 23
<210> 9
   <211> 18
   <212> DNA
   <213> Bacteriophage T7
<400> 9
   taatacgact cactatag 18
<210> 10
   <211> 23
   <212> DNA
   <213> Bacteriophage SP6
<220>
   <221> misc_feature
   <222> (22)..(22)
   <223> n is a, c, g, or t
<400> 10
   atttaggtga cactatagaa gng 23
<210> 11
   <211> 15
   <212> DNA
   <213> Cyanophage Syn5
<400> 11
   attgggcacc cgtaa 15
<210> 12
   <211> 41
   <212> DNA
   <213> Artificial Sequence
<220>
   <223> Adapter P1 forward
<400> 12
   ccactacgcc tccgctttcc tctctatggg cagtcggtga t 41
<210> 13
   <211> 43
   <212> DNA
   <213> Artificial Sequence
<220>
   <223> adapter p1 reverse
<400> 13
   atcaccgact gcccatagag aggaaagcgg aggcgtagtg gtt 43
<210> 14
   <211> 41
   <212> DNA
   <213> Artificial Sequence
<220>
   <223> barcode adapter A
<220>
   <221> misc_feature
   <222> (31)..(38)
   <223> n is a, c, g, or t
<400> 14
   ccatctcatc cctgcgtgtc tccgactcag nnnnnnnnga t 41
<210> 15
   <211> 43
   <212> DNA
   <213> Artificial Sequence
<220>
   <223> barcode adapter A reverse
<220>
   <221> misc_feature
   <222> (4)..(11)
   <223> n is a, c, g, or t
<400> 15
   atcnnnnnnn nctgagtcgg agacacgcag ggatgagatg gtt 43
<210> 16
   <211> 42
   <212> DNA
   <213> Artificial Sequence
<220>
   <223> Adapter-Pl-T forward
<400> 16
   ccactacgcc tccgctttcc tctctatggg cagtcggtga tt 42
<210> 17
   <211> 43
   <212> DNA
   <213> Artificial Sequence
<220>
   <223> Adpater-P1-T1 reverse
<400> 17
   atcaccgact gcccatagag aggaaagcgg aggcgtagtg gtt 43
<210> 18
   <211> 42
   <212> DNA
   <213> Artificial Sequence
<220>
   <223> Barcode Adapter A-T forward
<220>
   <221> misc_feature
   <222> (31)..(38)
   <223> n is a, c, g, or t
<400> 18
   ccatctcatc cctgcgtgtc tccgactcag nnnnnnnnga tt 42
<210> 19
   <211> 43
   <212> DNA
   <213> Artificial Sequence
<220>
   <223> Barcode Adapter A-T reverse
<220>
   <221> misc_feature
   <222> (4)..(11)
   <223> n is a, c, g, or t
<400> 19
   atcnnnnnnn nctgagtcgg agacacgcag ggatgagatg gtt 43
<210> 20
   <211> 41
   <212> DNA
   <213> Artificial Sequence
<220>
   <223> Full length primer P1
<400> 20
   ccactacgcc tccgctttcc tctctatggg cagtcggtga t 41
<210> 21
   <211> 30
   <212> DNA
   <213> Artificial Sequence
<220>
   <223> full length primer A
<400> 21
   ccatctcatc cctgcgtgtc tccgactcag 30
<210> 22
   <211> 19
   <212> DNA
   <213> Artificial Sequence
<220>
   <223> shortened primer minA
<400> 22
   ctgcgtgtct ccgactcag 19
<210> 23
   <211> 22
   <212> DNA
   <213> Artificial Sequence
<220>
   <223> shortened primer minP1
<400> 23
   ctctctatgg gcagtcggtg at 22
<210> 24
   <211> 42
   <212> DNA
   <213> Artificial Sequence
<220>
   <223> Barcode Adapter Ia+T forward
<220>
   <221> modified_base
   <222> (14)..(14)
   <223> I
<220>
   <221> misc_feature
   <222> (14)..(14)
   <223> n is a, c, g, or t
<220>
   <221> modified_base
   <222> (16)..(16)
   <223> I
<220>
   <221> misc_feature
   <222> (16)..(16)
   <223> n is a, c, g, or t
<220>
   <221> modified_base
   <222> (18)..(18)
   <223> I
<220>
   <221> misc_feature
   <222> (18)..(18)
   <223> n is a, c, g, or t
<220>
   <221> modified_base
   <222> (24)..(24)
   <223> I
<220>
   <221> misc_feature
   <222> (24)..(24)
   <223> n is a, c, g, or t
<220>
   <221> modified_base
   <222> (30)..(30)
   <223> I
<220>
   <221> misc_feature
   <222> (30)..(38)
   <223> n is a, c, g, or t
<400> 24
   ccatctcatc cctncntntc tccnactcan nnnnnnnnga tt 42
<210> 25
   <211> 43
   <212> DNA
   <213> Artificial Sequence
<220>
   <223> Barcode Adapter Ia+T reverse
<220>
   <221> misc_feature
   <222> (4)..(11)
   <223> n is a, c, g, or t
<220>
   <221> modified_base
   <222> (13)..(13)
   <223> I
<220>
   <221> misc_feature
   <222> (13)..(13)
   <223> n is a, c, g, or t
<220>
   <221> modified_base
   <222> (17)..(17)
   <223> I
<220>
   <221> misc_feature
   <222> (17)..(17)
   <223> n is a, c, g, or t
<220>
   <221> modified_base
   <222> (34)..(34)
   <223> I
<220>
   <221> misc_feature
   <222> (34)..(34)
   <223> n is a, c, g, or t
<220>
   <221> modified_base
   <222> (39)..(39)
   <223> I
<220>
   <221> misc_feature
   <222> (39)..(39)
   <223> n is a, c, g, or t
<220>
   <221> misc_feature
   <222> (42)..(43)
   <223> phosporothioate modification
<400> 25
   atcnnnnnnn nangagnagg agaaaagaag ggangagang gat 43
<210> 26
   <211> 42
   <212> DNA
   <213> Artificial Sequence
<220>
   <223> Barcode Adapter oligo IA+T
<220>
   <221> modified_base
   <222> (14)..(14)
   <223> I
<220>
   <221> misc_feature
   <222> (14)..(14)
   <223> n is a, c, g, or t
<220>
   <221> modified_base
   <222> (16)..(16)
   <223> I
<220>
   <221> misc_feature
   <222> (16)..(16)
   <223> n is a, c, g, or t
<220>
   <221> modified_base
   <222> (18)..(18)
   <223> I
<220>
   <221> misc_feature
   <222> (18)..(18)
   <223> n is a, c, g, or t
<220>
   <221> modified_base
   <222> (24)..(24)
   <223> I
<220>
   <221> misc_feature
   <222> (24)..(24)
   <223> n is a, c, g, or t
<220>
   <221> modified_base
   <222> (30)..(30)
   <223> I
<220>
   <221> misc_feature
   <222> (30)..(38)
   <223> n is a, c, g, or t
<400> 26
   ccatctcatc cctncntntc tccnactcan nnnnnnnnga tt 42
<210> 27
   <211> 43
   <212> DNA
   <213> Artificial Sequence
<220>
   <223> Barcode Adapter oligonucleotide IA'
<220>
   <221> misc_feature
   <222> (4)..(11)
   <223> n is a, c, g, or t
<220>
   <221> modified_base
   <222> (13)..(13)
   <223> I
<220>
   <221> misc_feature
   <222> (13)..(13)
   <223> n is a, c, g, or t
<220>
   <221> modified_base
   <222> (17)..(17)
   <223> I
<220>
   <221> misc_feature
   <222> (17)..(17)
   <223> n is a, c, g, or t
<220>
   <221> modified_base
   <222> (34)..(34)
   <223> I
<220>
   <221> misc_feature
   <222> (34)..(34)
   <223> n is a, c, g, or t
<220>
   <221> modified_base
   <222> (39)..(39)
   <223> I
<220>
   <221> misc_feature
   <222> (39)..(39)
   <223> n is a, c, g, or t
<400> 27
   atcnnnnnnn nangagnagg agaaaagaag ggangagang gat 43
<210> 28
   <211> 32
   <212> DNA
   <213> Artificial Sequence
<220>
   <223> Inosine Primer sequence for melting temperature calculation
<220>
   <221> modified_base
   <222> (2) .. (2)
   <223> I
<220>
   <221> misc_feature
   <222> (2)..(2)
   <223> n is a, c, g, or t
<220>
   <221> modified_base
   <222> (6)..(6)
   <223> I
<220>
   <221> misc_feature
   <222> (6)..(6)
   <223> n is a, c, g, or t
<220>
   <221> modified_base
   <222> (23)..(23)
   <223> I
<220>
   <221> misc_feature
   <222> (23)..(23)
   <223> n is a, c, g, or t
<220>
   <221> modified_base
   <222> (28)..(28)
   <223> I
<220>
   <221> misc_feature
   <222> (28)..(28)
   <223> n is a, c, g, or t
<400> 28
   angagnagga gaaaagaagg gangagangg at 32
<210> 29
   <211> 32
   <212> DNA
   <213> Artificial Sequence
<220>
   <223> Complement to Inosine Primer sequence
<400> 29
   atccctctcc tcccttcttt tctcctcctc ct 32
<210> 30
   <211> 30
   <212> DNA
   <213> Artificial Sequence
<220>
   <223> Converted standard to inosine primer sequence
<400> 30
   ccatctcatc cctgcgtgtc tccgactcag 30
<210> 31
   <211> 30
   <212> DNA
   <213> Artificial Sequence
<220>
   <223> Complement to converted standard
<400> 31
   ctgagtcgga gacacgcagg gatgagatgg 30
<210> 32
   <211> 32
   <212> DNA
   <213> Artificial Sequence
<220>
   <223> Primer A' sequence
<400> 32
   atccctctcc tcccttcttt tctcctactc ct 32
<210> 33
   <211> 32
   <212> DNA
   <213> Artificial Sequence
<220>
   <223> Primer A' complement
<400> 33
   aggagtagga gaaaagaagg gaggagaggg at 32
<210> 34
   <211> 41
   <212> DNA
   <213> Artificial Sequence
<220>
   <223> Full lenght primer P1 complement
<400> 34
   atcaccgact gcccatagag aggaaagcgg aggcgtagtg g 41
<210> 35
   <211> 23
   <212> DNA
   <213> Artificial Sequence
<220>
   <223> Delta5-P1
<400> 35
   cctctctatg ggcagtcggt gat 23
<210> 36
   <211> 23
   <212> DNA
   <213> Artificial Sequence
<220>
   <223> Delta5-P1 complement
<400> 36
   atcaccgact gcccatagag agg 23
<210> 37
   <211> 53
   <212> DNA
   <213> Artificial Sequence
<220>
   <223> Delta5-P1-A'
<400> 37
   cctctctatg ggcagtcggt gatccctctc ctcccttctt ttctcctact cct 53
<210> 38
   <211> 53
   <212> DNA
   <213> Artificial Sequence
<220>
   <223> Delta5-P1-A' complement
<400> 38
   aggagtagga gaaaagaagg gaggagaggg atcaccgact gcccatagag agg 53
<210> 39
   <211> 19
   <212> DNA
   <213> Escherichia coli
<400> 39
   agatgtgtat aagagacag 19
<210> 40
   <211> 17
   <212> DNA
   <213> Artificial Sequence
<220>
   <223> resulting sequence for 5'-extension for Tn5 adapter
<400> 40
   gcctccctcg cgccatc 17
<210> 41
   <211> 17
   <212> DNA
   <213> Artificial Sequence
<220>
   <223> resulting sequence for 5'-extension for Tn5 adapter
<400> 41
   gccttgccag cccgctc 17
<210> 42
   <211> 44
   <212> DNA
   <213> Artificial Sequence
<220>
   <223> Ada142dT-BC2 oligo for preparation of convertible Adapter IA+T with barcode
<220>
   <221> modified_base
   <222> (14)..(14)
   <223> I
<220>
   <221> misc_feature
   <222> (14)..(14)
   <223> n is a, c, g, or t
<220>
   <221> modified_base
   <222> (16)..(16)
   <223> I
<220>
   <221> misc_feature
   <222> (16)..(16)
   <223> n is a, c, g, or t
<220>
   <221> modified_base
   <222> (18)..(18)
   <223> I
<220>
   <221> misc_feature
   <222> (18)..(18)
   <223> n is a, c, g, or t
<220>
   <221> modified_base
   <222> (24)..(24)
   <223> I
<220>
   <221> misc_feature
   <222> (24)..(24)
   <223> n is a, c, g, or t
<220>
   <221> modified_base
   <222> (30)..(30)
   <223> I
<220>
   <221> misc_feature
   <222> (30)..(30)
   <223> n is a, c, g, or t
<400> 42
   ccatctcatc cctncntntc tccnactcan taaggagaac gatt 44
<210> 43
   <211> 45
   <212> DNA
   <213> Artificial Sequence
<220>
   <223> Ada142pos-BC2 oligo for preparation of convertible adapter IA+T
<220>
   <221> modified_base
   <222> (15)..(15)
   <223> I
<220>
   <221> misc_feature
   <222> (15)..(15)
   <223> n is a, c, g, or t
<220>
   <221> modified_base
   <222> (19)..(19)
   <223> I
<220>
   <221> misc_feature
   <222> (19)..(19)
   <223> n is a, c, g, or t
<220>
   <221> modified_base
   <222> (36)..(36)
   <223> I
<220>
   <221> misc_feature
   <222> (36)..(36)
   <223> n is a, c, g, or t
<220>
   <221> modified_base
   <222> (41)..(41)
   <223> I
<220>
   <221> misc_feature
   <222> (41)..(41)
   <223> n is a, c, g, or t
<220>
   <221> misc_feature
   <222> (44)..(45)
   <223> phosphorothioate modification
<400> 43
   atcgttctcc ttaangagna ggagaaaaga agggangaga nggat 45
<210> 44
   <211> 43
   <212> DNA
   <213> Artificial Sequence
<220>
   <223> template strand PF_Ad142v2BC4_r
<220>
   <221> modified_base
   <222> (16)..(16)
   <223> I
<220>
   <221> misc_feature
   <222> (16)..(16)
   <223> n is a, c, g, or t
<220>
   <221> modified_base
   <222> (18)..(18)
   <223> I
<220>
   <221> misc_feature
   <222> (18)..(18)
   <223> n is a, c, g, or t
<220>
   <221> modified_base
   <222> (21)..(21)
   <223> I
<220>
   <221> misc_feature
   <222> (21)..(21)
   <223> n is a, c, g, or t
<220>
   <221> modified_base
   <222> (24)..(24)
   <223> I
<220>
   <221> misc_feature
   <222> (24)..(24)
   <223> n is a, c, g, or t
<220>
   <221> modified_base
   <222> (29)..(29)
   <223> I
<220>
   <221> misc_feature
   <222> (29)..(29)
   <223> n is a, c, g, or t
<220>
   <221> modified_base
   <222> (32)..(32)
   <223> I
<220>
   <221> misc_feature
   <222> (32)..(32)
   <223> n is a, c, g, or t
<220>
   <221> modified_base
   <222> (37)..(37)
   <223> I
<220>
   <221> misc_feature
   <222> (37)..(37)
   <223> n is a, c, g, or t
<220>
   <221> modified_base
   <222> (42)..(42)
   <223> I
<220>
   <221> misc_feature
   <222> (42)..(42)
   <223> n is a, c, g, or t
<400> 44
   atcgatcttg gtactnantc nganacacnc anggatnaga tng 43
<210> 45
   <211> 45
   <212> DNA
   <213> Artificial Sequence
<220>
   <223> Primer binding strand PF_Ad142v2BC4_f)
<400> 45
   gccaatctaa tccatgagtg tatcagaata agtaccaaga tcgat 45
<210> 46
   <211> 32
   <212> DNA
   <213> Artificial Sequence
<220>
   <223> Amplification primer PF_ApI_prim
<400> 46
   gccaatctaa tccatgagtg tatcagaata ag 32

## Claims

1. A method of producing an asymmetrically tagged nucleic acid fragment, said method comprising the steps:
i. ligating a first adapter nucleic acid molecule to a template strand of a double-stranded nucleic acid fragment and a second adapter nucleic acid molecule to a complementary strand of said double-stranded nucleic acid fragment, wherein said first and said second adapter nucleic acid molecule are **characterized by** an identical base sequence and comprise a ligation site and a primer-binding region, and said primer-binding region comprises
a. at least one universal base and/or
b. at least one convertible base,
and wherein optionally said first adapter nucleic acid molecule and said second adapter nucleic acid molecule are double-stranded, and wherein optionally both strands of said first and said second adapter nucleic acid molecule are ligated to said double-stranded nucleic acid fragment;
ii. optionally converting said at least one convertible base comprised within said first and/or said second adapter nucleic acid molecule into an at least one converted base;
iii. obtaining a first antisense adapter and a second antisense adapter by performing a first extension step, wherein
a. said complementary strand is extended yielding said first antisense adapter ligated to said complementary strand, and said template strand is extended yielding a second antisense adapter ligated to said template strand, or
b. a primer is annealed to a strand of said second adapter nucleic acid molecule ligated to said template strand and extended yielding a nucleic acid strand comprising said primer, a nucleic acid sequence complementary to said template strand, and said first antisense adapter, and
a primer is annealed to a strand of said first adapter nucleic acid molecule ligated to said complementary strand and extended yielding a nucleic acid strand comprising said primer, a nucleic acid sequence complementary to said complementary strand, and said second antisense adapter,
wherein said first antisense adapter is complementary to said first adapter nucleic acid molecule and said second antisense adapter is complementary to said second adapter nucleic acid molecule except at positions opposite to said at least one universal, convertible or converted base comprised within said first and second adapter nucleic acid molecule, thereby yielding an asymmetrically tagged nucleic acid fragment.

2. The method according to claim 1, said method comprising the steps:
i. ligating a first adapter nucleic acid molecule to a template strand of a double-stranded nucleic acid fragment and a second adapter nucleic acid molecule to a complementary strand of said double-stranded nucleic acid fragment, wherein said first and said second adapter nucleic acid molecule are **characterized by** an identical base sequence and comprise a ligation site and a primer-binding region, and said primer-binding region comprises
- at least one universal base,
ii. obtaining a first antisense adapter and a second antisense adapter by performing a first extension step, wherein
- said complementary strand is extended yielding said first antisense adapter ligated to said complementary strand, and said template strand is extended yielding a second antisense adapter ligated to said template strand,
iii. obtaining a third antisense adapter and a fourth antisense adapter by performing a second extension step, wherein
• a primer is annealed to said second antisense adapter and extended yielding a nucleic acid strand comprising said primer, a nucleic acid complementary to said template strand, and a third antisense adapter,
• a primer is annealed to said first antisense adapter, and extended yielding a nucleic acid strand comprising said primer, a nucleic acid complementary to said complementary strand, and a fourth antisense adapter, and
• said third antisense adapter is complementary to said first adapter nucleic acid molecule and said second antisense adapter is complementary said second adapter nucleic acid molecule except at positions opposite to said at least one universal base comprised within said first and second adapter nucleic acid molecule, and said third and fourth antisense adapter are **characterized by** different base sequences, thereby yielding an asymmetrically tagged nucleic acid fragment, wherein said first extension step is performed by a first polymerase with a first base bias, and said second extension steps is performed by a second polymerase with a second base bias.

3. The method according to claim 1, said method comprising the steps:
i. ligating a first adapter nucleic acid molecule to a template strand of a double-stranded nucleic acid fragment and a second adapter nucleic acid molecule to a complementary strand of said double-stranded nucleic acid fragment, wherein said first and said second adapter nucleic acid molecule are **characterized by** an identical base sequence and comprise a ligation site and a primer-binding region, and said primer-binding region comprises
- at least one universal base,
and wherein said first adapter nucleic acid molecule and said second adapter nucleic acid molecule are double-stranded, and wherein both strands of said first and said second adapter nucleic acid molecule are ligated to said double-stranded nucleic acid fragment;
ii. obtaining a first antisense adapter and a second antisense adapter by performing a first extension step, wherein
- a primer is annealed to a strand of said second adapter nucleic acid molecule ligated to said template strand and extended yielding a nucleic acid strand comprising said primer, a nucleic acid sequence complementary to said template strand, and said first antisense adapter, and
a primer is annealed to a strand of said first adapter nucleic acid molecule ligated to said complementary strand and extended yielding a nucleic acid strand comprising said primer, a nucleic acid sequence complementary to said complementary strand, and said second antisense adapter,
wherein said first antisense adapter is complementary to said first adapter nucleic acid molecule and said second antisense adapter is complementary to said second adapter nucleic acid molecule except at positions opposite to said at least one universal base comprised within said first and second adapter nucleic acid molecule, thereby yielding an asymmetrically tagged nucleic acid fragment.

4. The method according to claim 1, said method comprising the steps:
i. ligating a first adapter nucleic acid molecule to a template strand of a double-stranded nucleic acid fragment and a second adapter nucleic acid molecule to a complementary strand of said double-stranded nucleic acid fragment, wherein said first and said second adapter nucleic acid molecule are **characterized by** an identical base sequence and comprise a ligation site and a primer-binding region, and said primer-binding region comprises
- at least one convertible base,
and wherein said first adapter nucleic acid molecule and said second adapter nucleic acid molecule are double-stranded, and wherein both strands of said first and said second adapter nucleic acid molecule are ligated to said double-stranded nucleic acid fragment;
ii. converting said at least one convertible base comprised with said first and/or said second adapter nucleic acid molecule into an at least one converted base;
iii. obtaining a first antisense adapter and a second antisense adapter by performing a first extension step, wherein
- a primer is annealed to a strand of said second adapter nucleic acid molecule ligated to said template strand and extended yielding a nucleic acid strand comprising said primer, a nucleic acid sequence complementary to said template strand, and said first antisense adapter, and
a primer is annealed to a strand of said first adapter nucleic acid molecule ligated to said complementary strand and extended yielding a nucleic acid strand comprising said primer, a nucleic acid sequence complementary to said complementary strand, and said second antisense adapter,
wherein said first antisense adapter is complementary to said first adapter nucleic acid molecule and said second antisense adapter is complementary to said second adapter nucleic acid molecule except at positions opposite to said at least one convertible or converted base comprised within said first and second adapter nucleic acid molecule, thereby yielding an asymmetrically tagged nucleic acid fragment, wherein said at least one convertible base and said at least one converted base pair with different bases.

5. The method according to any one of claims 1-3, wherein said at least one universal base is selected from inosine (hypoxanthine), xanthine, oxanine, 8-oxo-guanine, nebularine, 3-nitropyrrole, 5-nitroindole, 4-methylindole, 04-methyl-thymidine (04mT), 04-ethyl-thymidine (04eT), 6H,8H-3,4-dihydro-pyrimido[4,5-c][1,2]oxazin-7-one (P), N6-methoxy-2,6-30 diaminopurine (K), a 5-fluoroindole base, pyrrolidine, a dSpacer (1',2'-dideoxyribose) or an abasic site.

6. The method according to any one of claims 1 or 4, wherein said at least one convertible base is selected from 04-methyl-thymidine, 06-methyl-guanine, S4-methyl-thio-uridine, S4-methyl-thio-thymidine, S6-methyl-thio-guanine, N1-methyl-adenine, N3-methyl-adenine, C8-methyl-guanine, N2,3-etheno-guanine, 06-methyl-hypoxanthine, 04-ethylthymidine, 06-ethyl-guanine, S4-ethyl-thio-uridine, S4-ethyl-thio-thymidine, S6-ethyl-5 thio-guanine, N1- ethyl-adenine, N3-ethyl-adenine, C8-ethyl-guanine, 06-ethyl-hypoxanthine, S4-thio-uridine, S4-thiothymidine, S6-thio-guanine, 1-ethynyl-dSpacer and 1-thiol-dSpacer.

7. The method according to any one of claims 3-6, comprising
- said first extension step, and additionally
- a second extension step, wherein
• a primer is annealed to said second antisense adapter, and extended yielding a nucleic acid strand comprising said primer, a nucleic acid sequence complementary to said complementary strand, and a third antisense adapter and,
• a primer is annealed to said first antisense adapter and extended yielding a nucleic acid strand comprising said primer, a nucleic acid sequence complementary to said template strand, and a fourth antisense adapter, and
• said third antisense adapter is essentially complementary to said first adapter nucleic acid molecule and said fourth antisense adapter is essentially complementary said second adapter nucleic acid molecule except at positions opposite to said universal, convertible or converted bases comprised within said first and second adapter nucleic acid molecule and said third and fourth antisense adapter are **characterized by** different base sequences, thereby yielding an asymmetrically tagged nucleic acid fragment.

8. The method according to any of claims 1, 4, 6 or 7, wherein said conversion of said at least one convertible base comprised within said primer binding region of said first and/or said second adapter nucleic acid molecule is performed by an enzymatic, chemical or photochemical reaction.

9. The method according to any of the previous claims, wherein the ligation in step i. is performed by a ligase, a topoisomerase, a recombinase or a transposase.

10. The method according to any of the previous claims, wherein any of said adapter nucleic acids molecules comprises one or more of the following: a barcode, a recombination site, a topoisomerase recognition site, or a transposase recognition site.

11. The method according to any of the previous claims, wherein
- a primer anneals to said strand of said double stranded second adapter nucleic acid molecule ligated to said template strand and to said first and/or third antisense adapter nucleic acid molecule at different temperatures, particularly differing by at least 2°C, and/or
- a primer anneals to said strand of said double stranded first adapter nucleic acid molecule ligated to said complementary strand and to said second and/or fourth antisense adapter nucleic acid molecule at different temperatures, particularly differing by at least 2°C.

## Patentansprüche

1. Ein Verfahren zur Herstellung eines asymmetrisch markierten Nukleinsäurefragments, wobei das Verfahren die Schritte umfasst:
i. Ligation eines ersten Adapter-Nukleinsäuremoleküls an einen Matrizenstrang eines doppelsträngigen Nukleinsäurefragments und eines zweiten Adapter-Nukleinsäuremoleküls an einen komplementären Strang des doppelsträngigen Nukleinsäurefragments, wobei das erste und das zweite Adapter-Nukleinsäuremolekül durch eine identische Basensequenz gekennzeichnet sind und eine Ligationsstelle und eine Primer-bindende Region umfassen und die Primer-bindende Region umfasst
a. mindestens eine universelle Base und/oder
b. mindestens eine konvertierbare Base,
und wobei optional das erste Adapter-Nukleinsäuremolekül und das zweite Adapter-Nukleinsäuremolekül doppelsträngig sind, und wobei optional beide Stränge des ersten und des zweiten Adapter-Nukleinsäuremoleküls an das doppelsträngige Nukleinsäurefragment ligiert sind;
ii. optionales Konvertieren der mindestens einen konvertierbaren Base, die in dem ersten und/oder dem zweiten Adapter-Nukleinsäuremolekül enthalten ist, in mindestens eine konvertierte Base;
iii. Erhalten eines ersten Antisense-Adapters und eines zweiten Antisense-Adapters durch Ausführen eines ersten Verlängerungsschritts, wobei
a. der komplementäre Strang verlängert wird, was den ersten Antisense-Adapter ergibt, der an den komplementären Strang ligiert ist, und der Matrizenstrang verlängert wird, was einen zweiten Antisense-Adapter ergibt, der an den Matrizenstrang ligiert ist, oder
b. ein Primer an einen Strang des zweiten Adapter-Nukleinsäuremoleküls, das an den Matrizenstrang ligiert ist, angelagert und verlängert wird, wobei ein Nukleinsäurestrang erhalten wird, der den Primer, eine zu dem Matrizenstrang komplementäre Nukleinsäuresequenz und den ersten Antisense-Adapter umfasst, und
ein Primer an einen Strang des ersten Adapter-Nukleinsäuremoleküls, das an den komplementären Strang ligiert ist, angelagert und verlängert wird, wodurch ein Nukleinsäurestrang erhalten wird, der den Primer, eine zu dem komplementären Strang komplementäre Nukleinsäuresequenz und den zweiten Antisense-Adapter umfasst,
wobei der erste Antisense-Adapter komplementär zu dem ersten Adapter-Nukleinsäuremolekül ist und der zweite Antisense-Adapter komplementär zu dem zweiten Adapter-Nukleinsäuremolekül ist, außer an Positionen gegenüber der mindestens einen universellen, konvertierbaren oder konvertierten Base, die in dem ersten und zweiten Nukleinsäuremolekül des Adapters enthalten ist, wodurch ein asymmetrisch markiertes Nukleinsäurefragment erhalten wird.

2. Das Verfahren nach Anspruch 1, wobei das Verfahren die Schritte umfasst:
i. Ligation eines ersten Adapter-Nukleinsäuremoleküls an einen Matrizenstrang eines doppelsträngigen Nukleinsäurefragments und eines zweiten Adapter-Nukleinsäuremoleküls an einen komplementären Strang des doppelsträngigen Nukleinsäurefragments, wobei das erste und das zweite Adapter-Nukleinsäuremolekül durch eine identische Basensequenz gekennzeichnet sind und eine Ligationsstelle und eine Primer-bindende Region umfassen, und die Primer-bindende Region umfasst
- mindestens eine universelle Base,
ii. Erhalten eines ersten Antisense-Adapters und eines zweiten Antisense-Adapters durch Ausführen eines ersten Verlängerungsschritts, wobei
- der komplementäre Strang verlängert wird, was den ersten Antisense-Adapter ergibt, der an den komplementären Strang ligiert ist, und der Matrizenstrang verlängert wird, was einen zweiten Antisense-Adapter ergibt, der an den Matrizenstrang ligiert ist,
iii. Erhalten eines dritten Antisense-Adapters und eines vierten Antisense-Adapters durch Ausführen eines zweiten Verlängerungsschritts, wobei
• ein Primer an den zweiten Antisense-Adapter angelagert und verlängert wird, wobei ein Nukleinsäurestrang erhalten wird, der den Primer, eine zu dem Matrizenstrang komplementäre Nukleinsäure und einen dritten Antisense-Adapter umfasst,
• ein Primer an den ersten Antisense-Adapter angelagert und verlängert wird, wobei ein Nukleinsäurestrang erhalten wird, der den Primer, eine zu dem komplementären Strang komplementäre Nukleinsäure und einen vierten Antisense-Adapter umfasst, und
• der dritte Antisense-Adapter komplementär zu dem Nukleinsäuremolekül des ersten Adapters ist und der zweite Antisense-Adapter komplementär zu dem Nukleinsäuremolekül des zweiten Adapters ist, außer an Positionen gegenüber der mindestens einen universellen Base, die in dem ersten und zweiten Adapter-Nukleinsäuremolekül enthalten ist, und der dritte und vierte Antisense-Adapter durch unterschiedliche Basensequenzen gekennzeichnet sind, wodurch ein asymmetrisch markiertes Nukleinsäurefragment erhalten wird, wobei der erste Extensionsschritt durch eine erste Polymerase mit einer ersten Basenpräferenz und der zweite Extensionsschritt durch eine zweite Polymerase mit einer zweiten Basenpräferenz durchgeführt wird.

3. Das Verfahren nach Anspruch 1, wobei das Verfahren die Schritte umfasst:
i. Ligation eines ersten Adapter-Nukleinsäuremoleküls an einen Matrizenstrang eines doppelsträngigen Nukleinsäurefragments und eines zweiten Adapter-Nukleinsäuremoleküls an einen komplementären Strang des doppelsträngigen Nukleinsäurefragments, wobei das erste und das zweite Adapter-Nukleinsäuremolekül durch eine identische Basensequenz gekennzeichnet sind und eine Ligationsstelle und eine Primer-bindende Region umfassen und die Primer-bindende Region umfasst
- mindestens eine universelle Base,
und wobei das erste Adapter-Nukleinsäuremolekül und das zweite Adapter-Nukleinsäuremolekül doppelsträngig sind, und wobei beide Stränge des ersten und des zweiten Adapter-Nukleinsäuremoleküls an das doppelsträngige Nukleinsäurefragment ligiert sind;
ii. Erhalten eines ersten Antisense-Adapters und eines zweiten Antisense-Adapters durch Ausführen eines ersten Extensionsschritts, wobei
- ein Primer an einen Strang des zweiten Adapter-Nukleinsäuremoleküls, das an den Matrizenstrang ligiert ist, angelagert und verlängert wird, wodurch ein Nukleinsäurestrang entsteht, der den Primer, eine zu dem Matrizenstrang komplementäre Nukleinsäuresequenz und den ersten Antisense-Adapter umfasst, und
ein Primer an einen Strang des ersten Adapter-Nukleinsäuremoleküls, das an den komplementären Strang ligiert ist, angelagert und verlängert wird, wodurch ein Nukleinsäurestrang entsteht, der den Primer, eine zu dem komplementären Strang komplementäre Nukleinsäuresequenz und den zweiten Antisense-Adapter umfasst,
wobei der erste Antisense-Adapter komplementär zu dem Nukleinsäuremolekül des ersten Adapters ist und der zweite Antisense-Adapter komplementär zu dem Nukleinsäuremolekül des zweiten Adapters ist, außer an Positionen gegenüber der mindestens einen Universalbase, die in dem ersten und zweiten Nukleinsäuremolekül des Adapters enthalten ist, wodurch ein asymmetrisch markiertes Nukleinsäurefragment erhalten wird.

4. Das Verfahren nach Anspruch 1, wobei das Verfahren die Schritte umfasst:
i. Ligation eines ersten Adapter-Nukleinsäuremoleküls an einen Matrizenstrang eines doppelsträngigen Nukleinsäurefragments und eines zweiten Adapter-Nukleinsäuremoleküls an einen komplementären Strang des doppelsträngigen Nukleinsäurefragments, wobei das erste und das zweite Adapter-Nukleinsäuremoleküls durch eine identische Basensequenz gekennzeichnet sind und eine Ligationsstelle und eine Primer-bindende Region umfassen, und die Primer-bindende Region umfasst
- mindestens eine konvertierbare Base,
und wobei das erste Adapter-Nukleinsäuremolekül und das zweite Adapter-Nukleinsäuremolekül doppelsträngig sind, und wobei beide Stränge des ersten und des zweiten Adapter-Nukleinsäuremoleküls an das doppelsträngige Nukleinsäurefragment ligiert sind;
ii. Konvertieren der mindestens einen konvertierbaren Base, die in dem ersten und/oder dem zweiten Adapter-Nukleinsäuremolekül enthalten ist, in mindestens eine konvertierte Base;
iii. Erhalten eines ersten Antisense-Adapters und eines zweiten Antisense-Adapters durch Ausführen eines ersten Extensionsschritts, wobei
- ein Primer an einen Strang des zweiten Adapter-Nukleinsäuremoleküls, das an den Matrizenstrang ligiert ist, angelagert und verlängert wird, wodurch ein Nukleinsäurestrang entsteht, der den Primer, eine zu dem Matrizenstrang komplementäre Nukleinsäuresequenz und den ersten Antisense-Adapter umfasst, und
ein Primer an einen Strang des ersten Adapter-Nukleinsäuremoleküls, das an den komplementären Strang ligiert ist, angelagert und verlängert wird, wodurch ein Nukleinsäurestrang entsteht, der den Primer, eine zu dem komplementären Strang komplementäre Nukleinsäuresequenz und den zweiten Antisense-Adapter umfasst,
wobei der erste Antisense-Adapter komplementär zu dem ersten Adapter-Nukleinsäuremolekül ist und der zweite Antisense-Adapter komplementär zu dem zweiten Adapter-Nukleinsäuremolekül ist, außer an Positionen gegenüber der mindestens einen konvertierbaren oder konvertierten Base, die in dem ersten und zweiten Adapter-Nukleinsäuremolekül enthalten ist, wodurch ein asymmetrisch markiertes Nukleinsäurefragment erhalten wird, wobei die mindestens eine konvertierbare Base und die mindestens eine konvertierte Base mit unterschiedlichen Basen paaren.

5. Das Verfahren nach einem der Ansprüche 1-3, wobei die mindestens eine Universalbase ausgewählt ist aus Inosin (Hypoxanthin), Xanthin, Oxanin, 8-Oxo-Guanin, Nebularin, 3-Nitropyrrol, 5-Nitroindol, 4-Methylindol, O4-Methylthymidin (04mT), O4-Ethylthymidin (04eT), 6H,8H-3,4-Dihydro-pyrimido[4,5-c][1,2]oxazin-7-on (P), N6-Methoxy-2,6-30-diaminopurin (K), eine 5-Fluorindol-Base, Pyrrolidin, ein dSpacer (1',2'-Dideoxyribose) oder eine abasische Stelle.

6. Das Verfahren nach einem der Ansprüche 1 oder 4, wobei die mindestens eine konvertierbare Base ausgewählt ist aus O4-Methylthymidin, O6-Methylguanin, S4-Methylthiouridin, S4-Methylthiothymidin, S6-Methylthioguanin, N1-Methyladenin, N3-Methyladenin, C8-Methylguanin, N2,3-Ethenoguanin, O6-Methyl-Hypoxanthin, 04-Ethylthymidin, O6-Ethyl-Guanin, S4-Ethyl-Thio-Uridin, S4-Ethyl-Thio-Thymidin, S6-Ethyl-5-Thio-Guanin, N1-Ethyl-Adenin, N3-Ethyl-adenin, C8-Ethyl-guanin, 06-Ethylhypoxanthin, S4-Thio-uridin, S4-Thiothymidin, S6-thio-guanin, 1-Ethinyl-dSpacer und 1-Thiol-dSpacer.

7. Das Verfahren nach einem der Ansprüche 3-6, umfassend
- den ersten Extensionsschritt, und zusätzlich
- einen zweiten Extensionsschritt, wobei
▪ ein Primer an den zweiten Antisense-Adapter angelagert und verlängert wird, wobei ein Nukleinsäurestrang erhalten wird, der den Primer, eine zu dem komplementären Strang komplementäre Nukleinsäuresequenz und einen dritten Antisense-Adapter umfasst, und
▪ ein Primer an den ersten Antisense-Adapter angelagert und verlängert wird, wobei ein Nukleinsäurestrang erhalten wird, der den Primer, eine zu dem Matrizenstrang komplementäre Nukleinsäuresequenz und einen vierten Antisense-Adapter umfasst, und
▪ der dritte Antisense-Adapter im Wesentlichen komplementär zu dem Nukleinsäuremolekül des ersten Adapters ist und der vierte Antisense-Adapter im Wesentlichen komplementär zu dem Nukleinsäuremolekül des zweiten Adapters ist, außer an Positionen gegenüber den universellen, konvertierbaren oder konvertierten Basen, die in dem ersten und zweiten Nukleinsäuremolekül des Adapters enthalten sind, und der dritte und vierte Antisense-Adapter durch unterschiedliche Basensequenzen gekennzeichnet sind, wodurch ein asymmetrisch markiertes Nukleinsäurefragment erhalten wird.

8. Das Verfahren nach einem der Ansprüche 1, 4, 6 oder 7, wobei die Konvertierung der mindestens einen konvertierbaren Base, die in der Primer-bindenden Region des ersten und/oder des zweiten Adapter-Nukleinsäuremoleküls enthalten ist, durch eine enzymatische, chemische oder photochemische Reaktion durchgeführt wird.

9. Das Verfahren nach einem der vorstehenden Ansprüche, wobei die Ligation in Schritt i. durch eine Ligase, eine Topoisomerase, eine Rekombinase oder eine Transposase durchgeführt wird.

10. Das Verfahren nach einem der vorhergehenden Ansprüche, wobei eines der Adapter-Nukleinsäuren-Moleküle einen oder mehrere der folgenden Bestandteile enthält:
einen Barcode, eine Rekombinationsstelle, eine Topoisomerase-Erkennungsstelle oder eine Transposase-Erkennungsstelle.

11. Das Verfahren nach einem der vorhergehenden Ansprüche, wobei
- ein Primer an den Strang des doppelsträngigen zweiten Adapter-Nukleinsäuremoleküls, das an den Matrizenstrang ligiert ist, und an das erste und/oder dritte Antisense-Adapter-Nukleinsäuremolekül bei verschiedenen Temperaturen anlagert, die sich insbesondere um mindestens 2°C unterscheiden, und/oder
- ein Primer an den Strang des doppelsträngigen ersten Adapter-Nukleinsäuremoleküls, das an den komplementären Strang ligiert ist, und an das zweite und/oder vierte Antisense-Adapter-Nukleinsäuremolekül bei verschiedenen Temperaturen anlagert, die sich insbesondere um mindestens 2°C unterscheiden.

## Revendications

1. Procédé de production d'un fragment d'acide nucléique à marquage asymétrique, ledit procédé comprenant les étapes consistant à :
i. ligaturer une première molécule d'acide nucléique adaptatrice à un brin de matrice d'un fragment d'acide nucléique double brin et une deuxième molécule d'acide nucléique adaptatrice à un brin complémentaire dudit fragment d'acide nucléique double brin, dans lequel ladite première et ladite deuxième molécules d'acide nucléique adaptatrices sont **caractérisées par** une séquence de bases identique et comprennent un site de ligature et une région de fixation d'amorce, et ladite région de fixation d'amorce comprend
a. au moins une base universelle et/ou
b. au moins une base convertible,
et dans lequel en option ladite première molécule d'acide nucléique adaptatrice et ladite deuxième molécule d'acide nucléique adaptatrice sont à double brin, et dans lequel en option les deux brins de ladite première et ladite deuxième molécules d'acide nucléique adaptatrices sont ligaturés audit fragment d'acide nucléique double brin ;
ii. en option convertir ladite au moins une base convertible composée de ladite première et/ou ladite deuxième molécule d'acide nucléique adaptatrice en au moins une base convertie ;
iii. obtenir un premier adaptateur antisens et un deuxième adaptateur antisens en réalisant une première étape d'extension, dans lequel
a. ledit brin complémentaire est étendu en donnant ledit premier adaptateur antisens ligaturé audit brin complémentaire, et ledit brin de matrice est étendu en donnant un deuxième adaptateur antisens ligaturé audit brin de matrice, ou
b. une amorce est annelée à un brin de ladite deuxième molécule d'acide nucléique adaptatrice ligaturée audit brin de matrice et étendue en donnant un brin d'acide nucléique comprenant ladite amorce, une séquence d'acide nucléique complémentaire audit brin de matrice, et ledit premier adaptateur antisens, et
une amorce est annelée à un brin de ladite première molécule d'acide nucléique adaptatrice ligaturée audit brin complémentaire et étendue en donnant un brin d'acide nucléique comprenant ladite amorce, une séquence d'acide nucléique complémentaire audit brin complémentaire, et ledit deuxième adaptateur antisens,
dans lequel ledit premier adaptateur antisens est complémentaire à ladite première molécule d'acide nucléique adaptatrice et ledit deuxième adaptateur antisens est complémentaire à ladite deuxième molécule d'acide nucléique adaptatrice sauf à des positions opposées à ladite au moins une base universelle, convertible ou convertie comprise au sein desdites première et deuxième molécules d'acide nucléique adaptatrices, donnant de ce fait un fragment d'acide nucléique à marquage asymétrique.

2. Procédé selon la revendication 1, ledit procédé comprenant les étapes consistant à :
i. ligaturer une première molécule d'acide nucléique adaptatrice à un brin de matrice d'un fragment d'acide nucléique double brin et une deuxième molécule d'acide nucléique adaptatrice à un brin complémentaire dudit fragment d'acide nucléique double brin, dans lequel ladite première et ladite deuxième molécules d'acide nucléique adaptatrices sont **caractérisées par** une séquence de bases identique et comprennent un site de ligature et une région de fixation d'amorce, et ladite région de fixation d'amorce comprend
- au moins une base universelle,
ii. obtenir un premier adaptateur antisens et un deuxième adaptateur antisens en réalisant une première étape d'extension, dans lequel
- ledit brin complémentaire est étendu en donnant ledit premier adaptateur antisens ligaturé audit brin complémentaire, et ledit brin de matrice est étendu en donnant un deuxième adaptateur antisens ligaturé audit brin de matrice,
iii. obtenir un troisième adaptateur antisens et un quatrième adaptateur antisens en réalisant une seconde étape d'extension, dans lequel
• une amorce est annelée audit deuxième adaptateur antisens et étendue en donnant un brin d'acide nucléique comprenant ladite amorce, un acide nucléique complémentaire audit brin de matrice, et un troisième adaptateur antisens,
• une amorce est annelée audit premier adaptateur antisens et étendue en donnant un brin d'acide nucléique comprenant ladite amorce, un acide nucléique complémentaire audit brin complémentaire, et un quatrième adaptateur antisens, et
• ledit troisième adaptateur antisens est complémentaire à ladite première molécule d'acide nucléique adaptatrice et ledit deuxième adaptateur antisens est complémentaire à ladite deuxième molécule d'acide nucléique adaptatrice sauf à des positions opposées à ladite au moins une base universelle comprise au sein desdites première et deuxième molécules d'acide nucléique adaptatrices, et lesdits troisième et quatrième adaptateurs antisens sont **caractérisés par** différentes séquences de bases, donnant de ce fait un fragment d'acide nucléique à marquage asymétrique, dans lequel ladite première étape d'extension est réalisée par une première polymérase avec une première polarisation de base, et ladite seconde étape d'extension est par une seconde polymérase avec une seconde préférence de base.

3. Procédé selon la revendication 1, ledit procédé comprenant les étapes consistant à :
i. ligaturer une première molécule d'acide nucléique adaptatrice à un brin de matrice d'un fragment d'acide nucléique double brin et une deuxième molécule d'acide nucléique adaptatrice à un brin complémentaire dudit fragment d'acide nucléique double brin, dans lequel ladite première et ladite deuxième molécules d'acide nucléique adaptatrices sont **caractérisées par** une séquence de bases identique et comprennent un site de ligature et une région de fixation d'amorce, et ladite région de fixation d'amorce comprend
- au moins une base universelle,
et dans lequel ladite première molécule d'acide nucléique adaptatrice et ladite deuxième molécule d'acide nucléique adaptatrice sont à double brin, et dans lequel les deux brins de ladite première et ladite deuxième molécules d'acide nucléique adaptatrices sont ligaturés audit fragment d'acide nucléique double brin ;
ii. obtenir un premier adaptateur antisens et un deuxième adaptateur antisens en réalisant une première étape d'extension, dans lequel
- une amorce est annelée à un brin de ladite deuxième molécule d'acide nucléique adaptatrice ligaturée audit brin de matrice et étendue en donnant un brin d'acide nucléique comprenant ladite amorce, une séquence d'acide nucléique complémentaire audit brin de matrice, et ledit premier adaptateur antisens, et
une amorce est annelée à un brin de ladite première molécule d'acide nucléique adaptatrice ligaturée audit brin complémentaire et étendue en donnant un brin d'acide nucléique comprenant ladite amorce, une séquence d'acide nucléique complémentaire audit brin complémentaire, et ledit deuxième adaptateur antisens,
dans lequel ledit premier adaptateur antisens est complémentaire à ladite première molécule d'acide nucléique adaptatrice et ledit deuxième adaptateur antisens est complémentaire à ladite deuxième molécule d'acide nucléique adaptatrice sauf à des positions opposées à ladite au moins une base universelle comprise au sein desdites première et deuxième molécules d'acide nucléique adaptatrices, donnant de ce fait un fragment d'acide nucléique à marquage asymétrique.

4. Procédé selon la revendication 1, ledit procédé comprenant les étapes consistant à :
i. ligaturer une première molécule d'acide nucléique adaptatrice à un brin de matrice d'un fragment d'acide nucléique double brin et une deuxième molécule d'acide nucléique adaptatrice à un brin complémentaire dudit fragment d'acide nucléique double brin, dans lequel ladite première et ladite deuxième molécules d'acide nucléique adaptatrices sont **caractérisées par** une séquence de bases identique et comprennent un site de ligature et une région de fixation d'amorce, et ladite région de fixation d'amorce comprend
- au moins une base convertible,
et dans lequel ladite première molécule d'acide nucléique adaptatrice et ladite deuxième molécule d'acide nucléique adaptatrice sont à double brin, et dans lequel les deux brins de ladite première et ladite deuxième molécules d'acide nucléique adaptatrices sont ligaturés audit fragment d'acide nucléique double brin ;
ii. convertir ladite au moins une base convertible composée de ladite première et/ou ladite deuxième molécule d'acide nucléique adaptatrice en au moins une base convertie ;
iii. obtenir un premier adaptateur antisens et un deuxième adaptateur antisens en réalisant une première étape d'extension, dans lequel
- une amorce est annelée à un brin de ladite deuxième molécule d'acide nucléique adaptatrice ligaturée audit brin de matrice et étendue en donnant un brin d'acide nucléique comprenant ladite amorce, une séquence d'acide nucléique complémentaire audit brin de matrice, et ledit premier adaptateur antisens, et
une amorce est annelée à un brin de ladite première molécule d'acide nucléique adaptatrice ligaturée audit brin complémentaire et étendue en donnant un brin d'acide nucléique comprenant ladite amorce, une séquence d'acide nucléique complémentaire audit brin complémentaire, et ledit deuxième adaptateur antisens,
dans lequel ledit premier adaptateur antisens est complémentaire à ladite première molécule d'acide nucléique adaptatrice et ledit deuxième adaptateur antisens est complémentaire à ladite deuxième molécule d'acide nucléique adaptatrice sauf à des positions opposées à ladite au moins une base convertible ou convertie comprise au sein desdites première et deuxième molécules d'acide nucléique adaptatrices, donnant de ce fait un fragment d'acide nucléique à marquage asymétrique, dans lequel ladite au moins une base convertible et ladite au moins une base convertie s'apparient avec différentes bases.

5. Procédé selon l'une quelconque des revendications 1 à 3, dans lequel ladite au moins une base universelle est sélectionnée parmi l'inosine (hypoxanthine), la xanthine, l'oxanine, la 8-oxo-guanine, la nébularine, le 3-nitropyrrole, le 5-nitroindole, le 4-méthylindole, la O4-méthyl-thymidine (O4mT), la O4-éthyl-thymidine (O4eT), la 6H,8H-3,4-dihydro-pyrimido[4,5-c][1,2]oxazin-7-one (P), la N6-méthoxy-2,6-30 diaminopurine (K), une base de 5-fluoroindole, la pyrrolidine, un dSpacer (1',2'-didésoxyribose) ou un site abasique.

6. Procédé selon l'une quelconque des revendications 1 à 4, dans lequel ladite au moins une base convertible est sélectionnée parmi la O4-méthyl-thymidine, la O6-méthyl-guanine, la S4-méthyl-thio-uridine, la S4-méthyl-thio-thymidine, la S6-méthyl-thio-guanine, la N1-méthyl-adénine, la N3-méthyl-adénine, la C8-méthyl-guanine, la N2,3-éthéno-guanine, la O6-méthyl-hypoxanthine, la O4-éthylthymidine, la O6-éthyl-guanine, la S4-éthyl-thio-uridine, la S4-éthyl-thio-thymidine, la S6-éthyl-5 thio-guanine, la N1-éthyl-adénine, la N3-éthyl-adénine, la C8-éthyl-guanine, la O6-éthyl-hypoxanthine, la S4-thio-uridine, la S4-thiothymidine, la S6-thio-guanine, le 1-éthynyl-dSpacer et le 1-thiol-dSpacer.

7. Procédé selon l'une quelconque des revendications 3 à 6, comprenant
- ladite première étape d'extension, et en outre
- une seconde étape d'extension, dans lequel
• une amorce est annelée audit deuxième adaptateur antisens et étendue en donnant un brin d'acide nucléique comprenant ladite amorce, un acide nucléique complémentaire audit brin complémentaire, et un troisième adaptateur antisens, et
• une amorce est annelée audit premier adaptateur antisens et étendue en donnant un brin d'acide nucléique comprenant ladite amorce, un acide nucléique complémentaire audit brin de matrice, et un quatrième adaptateur antisens, et
• ledit troisième adaptateur antisens est essentiellement complémentaire à ladite première molécule d'acide nucléique adaptatrice et ledit quatrième adaptateur antisens est essentiellement complémentaire à ladite deuxième molécule d'acide nucléique adaptatrice sauf à des positions opposées auxdites bases universelles, convertibles ou converties comprises au sein desdites première et deuxième molécules d'acide nucléique adaptatrices, et lesdits troisième et quatrième adaptateurs antisens sont **caractérisés par** différentes séquences de bases, donnant de ce fait un fragment d'acide nucléique à marquage asymétrique.

8. Procédé selon l'une quelconque des revendications 1, 4, 6 ou 7, dans lequel ladite conversion de ladite au moins une base convertible comprise au sein de ladite région de fixation d'amorce de ladite première et/ou ladite deuxième molécule d'acide nucléique adaptatrice est réalisée par une réaction enzymatique, chimique ou photochimique.

9. Procédé selon l'une quelconque des revendications précédentes, dans lequel la ligature à l'étape i. est réalisée par une ligase, une topoisomérase, une recombinase ou une transposase.

10. Procédé selon l'une quelconque des revendications précédentes, dans lequel l'une quelconque desdites molécules d'acide nucléique adaptatrices comprend un ou plusieurs des éléments suivants : un code-barres, un site de recombinaison, un site de reconnaissance de topoisomérase ou un site de reconnaissance de transposase.

11. Procédé selon l'une quelconque des revendications précédentes, dans lequel
- une amorce s'annèle audit brin de ladite deuxième molécule d'acide nucléique adaptatrice double brin ligaturée audit brin de matrice et à ladite première et/ou troisième molécule d'acide nucléique adaptatrice antisens à différentes températures, différant notamment d'au moins 2°C, et/ou
- une amorce s'annèle audit brin de ladite première molécule d'acide nucléique adaptatrice double brin ligaturée audit brin complémentaire et à ladite deuxième et/ou quatrième molécule d'acide nucléique adaptatrice antisens à différentes températures, différant notamment d'au moins 2°C.
